# EUROPEAN PATENT SPECIFICATION

(11) **EP 3 516 056 B1**
(45) Date of publication and mention of the grant of the patent: **27.11.2024**
(21) Application number: 17748410.2
(22) Date of filing: 17.07.2017
(51) Int. Cl.: C12N 15/10, C12N 15/63

(54) **A GUIDE-RNA EXPRESSION SYSTEM FOR A HOST CELL**
EIN GUIDE-RNS EXPRESSIONSSYSTEM FÜR EINE WIRTSZELLE
UN SYSTÈME D'EXPRESSION ARN-GUIDE POUR UNE CELLULE HÔTE

(30) Priority: 23.09.2016 US 201662399127 P
(43) Date of publication of application: 31.07.2019
(73) Proprietor: DSM IP Assets B.V., 6221 BE Maastricht (NL); Massachusetts Institute of Technology, Cambridge, MA 02139 (US)
(72) Inventor: YOUNG, Eric, Mosher, Maynard, MA 01754 (US); GHODASARA, Amar, Somerville, MA 02143 (US); VERWAAL, René, 6100 AA Echt (NL); ROUBOS, Johannes, Andries, 6100 AA Echt (NL); GIELESEN, Bianca, Elisabeth, Maria, 6100 AA Echt (NL); VONK, Brenda, 6100 AA Echt (NL); NIELSEN, Alec, A.K., Cambridge, MA 02139 (US); VOIGT, Christopher, Ashby, Belmont, MA 02478 (US)
(74) Representative: dsm-firmenich IP
(86) International application number: PCT/EP2017/068014
(87) International publication number: WO 2017/216392

(56) References cited:
- WO-A1-2014/093701
- WO-A1-2014/204724
- WO-A1-2014/204725
- WO-A1-2015/089351
- WO-A1-2016/110453
- WO-A2-2014/093622
- WO-A2-2015/089419
- CN-A- 104 651 399
- US-A1- 2016 032 295
- SOMAYEH ENAYATI ET AL: "T7-RNA polymerase dependent RNAi system in Aspergillus fumigatus : a proof of concept study", FEMS MICROBIOLOGY LETTERS, vol. 363, no. 5, 5 February 2016 (2016-02-05), pages fnw029, XP055767929, DOI: 10.1093/femsle/fnw029
- BIRGIT HOBL ET AL: "Bacteriophage T7 RNA polymerase-based expression in Pichia pastoris", PROTEIN EXPRESSION AND PURIFICATION., vol. 92, no. 1, 1 November 2013 (2013-11-01), SAN DIEGO, CA., pages 100 - 104, XP055767933, ISSN: 1046-5928, DOI: 10.1016/j.pep.2013.09.004
- JORGENSEN ELLEN D ET AL: "Specific Contacts between the Bacteriophage T3, T7 and SP6 RNA Prolymerases and Their Promoters", 1 January 1991 (1991-01-01), pages 645 - 651, XP093067999, Retrieved from the Internet <URL:https://www.jbc.org/article/S0021-9258(18)52483-2/pdf> [retrieved on 20230727]
- NIE YU ET AL: "Development of a bacterial-based negative selection system for rapid screening of active single guide RNAs", BIOTECHNOLOGY LETTERS, SPRINGER NETHERLANDS, NL, vol. 39, no. 3, 17 November 2016 (2016-11-17), pages 351 - 358, XP036148654, ISSN: 0141-5492, [retrieved on 20161117], DOI: 10.1007/S10529-016-2259-0
- LEI S. QI ET AL: "Repurposing CRISPR as an RNA-Guided Platform for Sequence-Specific Control of Gene Expression", CELL, vol. 152, no. 5, 28 February 2013 (2013-02-28), US, pages 1173 - 1183, XP055346792, ISSN: 0092-8674, DOI: 10.1016/j.cell.2013.02.022
- LUKE A. GILBERT ET AL: "CRISPR-Mediated Modular RNA-Guided Regulation of Transcription in Eukaryotes", CELL, vol. 154, no. 2, 1 July 2013 (2013-07-01), US, pages 442 - 451, XP055321615, ISSN: 0092-8674, DOI: 10.1016/j.cell.2013.06.044
- DIDOVYK ANDRIY ET AL: "Transcriptional regulation with CRISPR-Cas9: principles, advances, and applications", CURRENT OPINION IN BIOTECHNOLOGY, LONDON, GB, vol. 40, 23 June 2016 (2016-06-23), pages 177 - 184, XP029669926, ISSN: 0958-1669, DOI: 10.1016/J.COPBIO.2016.06.003
- J. E. DICARLO ET AL: "Genome engineering in Saccharomyces cerevisiae using CRISPR-Cas systems", NUCLEIC ACIDS RESEARCH, vol. 41, no. 7, 4 March 2013 (2013-03-04), pages 4336 - 4343, XP055086617, ISSN: 0305-1048, DOI: 10.1093/nar/gkt135

## Description

### Field

The present invention relates to the field of molecular biology and cell biology. More specifically, the present invention relates to a guide-RNA expression system for a host cell.

### Background

An RNA-guided nuclease system, from which the best known is the CRISPR/Cas9 system, is a powerful tool that has been leveraged for genome editing and gene regulation. This tool requires the expression of the Cas9 protein and a guide-RNA (gRNA or sgRNA) that enables Cas9 to target a specific sequence of DNA. In e.g. eukaryotic host systems, the guide-RNA is often expressed from RNA polymerase III (POLIII) promoters that recruit endogenous RNA polymerase III for transcription, which is an RNA polymerase that generates guide-RNAs without a 5' cap. Others have used RNA polymerase II (POLII) promoters in combinations with a ribozyme in order to produce guide-RNAs without a 5' cap (uncapped RNA).

WO 2014/093701, WO2014/204724, WO 2014/093622, WO 2014/204725, WO 2015/089419, and WO 2015/089351 mention in a hypothetical embodiment and prophetic example (except for WO 2015/089351), the use of T7 promoter for expressing of a gRNA in a human cell, but are silent on a yeast cell or a filamentous fungal cell. US 2016/032295 mentions in a hypothetical embodiment, the use of an SP6 or T7 promoter for expressing of a gRNA in a human cell, but is silent on the type of host cell and is thus silent on a yeast cell or a filamentous fungal cell. Jorgensen et al (1991; The Journal of Biological Chemistry, Vol. 266, No. 1, Issue of January 5, pp. 645-651) relates to background knowledge on T3, T7 and SP6 RNA polymerases. However, the state of the art expression of a guide-RNA requires complex and bulky expression cassettes and lacks straightforward tuning of the amount of guide-RNA. In addition, *in vitro* transcribed guide-RNA's can be introduced in a host cell. However, the transient nature of such an introduction limits the use of this method for genome editing and genome regulation. There is thus a continuing urge to develop improved and simplified expression systems for guide-RNA's within a cell.

### Summary

The embodiments of the invention are set forward in the claims herein.

Specifically, the invention provides for the use of a single-subunit DNA-dependent RNA polymerase selected from a T3, SP6, K11 or T7 RNA polymerase, for the expression within a fungal cell of a guide-RNA for an RNA-guided nuclease system, wherein the guide-RNA is encoded by a polynucleotide that is operably linked to a viral single-subunit DNA-dependent RNA polymerase promoter selected from a T3, SP6, K11 or T7 RNA polymerase promoter and wherein the fungal cell is a yeast cell or a filamentous fungus cell.

The invention further provides for a method for expression within a fungal cell of a guide-RNA for an RNA-guided nuclease system, wherein the guide-RNA is encoded by a polynucleotide that is operably linked to a viral single-subunit DNA-dependent RNA polymerase promoter selected from a T3, SP6, K11 or T7 RNA polymerase promoter, wherein transcription of the guide-RNA is performed by a viral single-subunit DNA-dependent RNA polymerase selected from aT3, SP6, K11 or T7 RNA polymerase, and wherein the fungal cell is a yeast cell or a filamentous fungus cell.

The invention further provides for a composition comprising the fungal cell, the RNA polymerase and the guide-RNA encoding polynucleotide operably linked to the promoter, according to the invention. The invention further provides for a fungal cell comprising at least the RNA polymerase and the guide-RNA encoding polynucleotide operably linked to the promoter, according to the invention, said cell preferably being capable of producing a compound of interest, wherein the fungal cell is a yeast cell or a filamentous fungus cell.

The invention further provides for a method for the production of a compound of interest comprising culturing the cell according to the invention under conditions conducive to the production of the compound of interest and, optionally, purifying or isolating said compound of interest.

### Brief description of the drawings

**Figure 1** depicts a graphical representation of the integrated green fluorescent protein (GFP) reporter, hygromycin B (HygB) selection marker and dCas9-Mxi1 (dCas9-Mxi) expression unit. dCas9-Mxi1 is expressed from a galactose-induced (GAL) promoter.
**Figure 2** depicts the map of the integrated green fluorescent protein (GFP) reporter, hygromycin B (HygB) selection marker and dCas9-Mxi1 (dCas9-Mxi) / T7-RNA polymerase (T7-RNAP) expression units. A 2A viral peptide (2A) is used to co-express dCas9-Mxi1 and T7-RNA polymerase from a galactose-induced (GAL) promoter.
**Figure 3** depicts the vector map of multicopy (2 micron) vector pRN1120-AG1. A nourseothricin (NatMX) and an ampicillin (ampR) marker are present on the vector. The vector contains the SNR52 promoter (SNR52p) that drives gRNA expression. The 20 nucleotide (nt) guide-sequence (genomic target sequence) and gRNA structural component are shown. The guide RNA sequence is followed by the SUP4 terminator (SUP4 3' flanking region).
**Figure 4** depicts the vector map of multicopy (2 micron) vector pAG701. A nourseothricin (NatMX) and an ampicillin (ampR) marker are present on the vector. The vector contains the T7 promoter that drives gRNA expression. The 20-nt guide-sequence (genomic target sequence) and gRNA structural component are shown. The gRNA sequence is followed by a 3' self-cleaving (self-processing) ribozyme (HDV) and a T7 terminator.
**Figure 5** depicts the repression efficiency of a SNR52-produced gRNA (scrambled (SNR52-scrambled) and non-scrambled (SNR52-target 3)) compared to a T7-produced gRNA (scrambled (T7-scrambled) and non-scrambled (T7)). The average and standard deviation of three individual experiments is depicted.
**Figure 6** depicts cell density (optical density measured at 600 nm) after galactose induction for all tested strains: SNR52-produced gRNA (scrambled (SNR52-scrambled) and non-scrambled (SNR52)), T7-produced gRNA (scrambled (T7-scrambled) and non-scrambled (T7)). The average and standard deviation of three individual experiments is depicted.
**Figure 7** depicts the cell populations of strains with SNR52-produced targeting and scrambled gRNAs.
**Figure 8** depicts the cell populations of strains with T7-produced targeting and scrambled gRNAs.
**Figure 9** depicts the repression efficiency of gRNAs produced from T7 promoters of varying strengths (T7-high, T7-medium, T7-low and T7-scrambled). The average and standard deviation of three individual experiments is depicted.
**Figure 10** depicts the vector map of single copy (CEN/ARS) vector pCSN061 expressing CAS9 codon pair optimized for expression in S. *cerevisiae.* A KanMX marker is present on the vector.
**Figure 11** depicts the vector map of multicopy (2 micron) vector pRN1120. A NatMX marker is present on the vector.

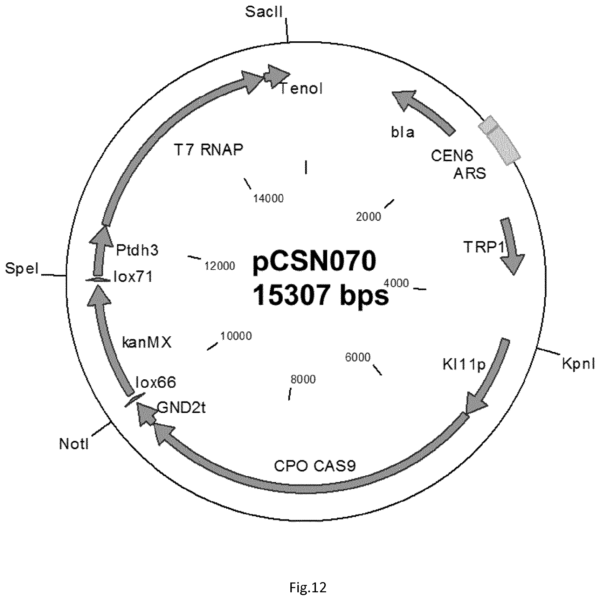

**Figure 12** depicts the vector map of single copy (CEN/ARS) vector pCSN070 expressing CAS9 codon pair optimized and T7 RNAP under control of the SbTDH3 promoter for expression in S. *cerevisiae.* A KanMX marker is present on the vector.

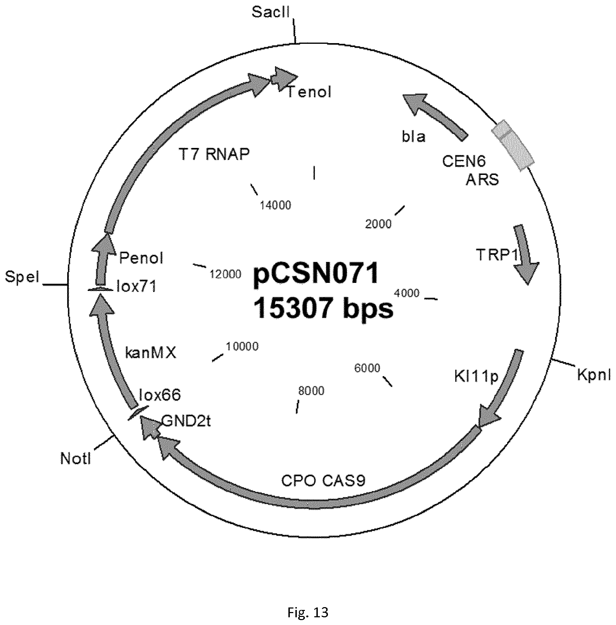

**Figure 13** depicts the vector map of single copy (CEN/ARS) vector pCSN071 expressing CAS9 codon pair optimized and T7 RNAP under control of the enol promoter for expression in S. *cerevisiae.* A KanMX marker is present on the vector.
**Figure 14** depicts the plasmid map of TOPO donor DNA fwnA
**Figure 15** depicts the plasmid map of BG-AMA17
**Figure 16** depicts the plasmid map of BG-AMA18
**Figure 17** depicts the plasmid map of BG-AMA19

### Description of the sequences

SEQ ID NO: 1 sets out the forward primer sequence used to amplify the 5' piece for integration into the INT1 locus.
SEQ ID NO: 2 sets out the reverse primer sequence used to amplify the 5' piece for integration into the INT1 locus.
SEQ ID NO: 3 sets out the forward primer sequence used to amplify the 3' piece for integration into the INT1 locus.
SEQ ID NO: 4 sets out the reverse primer sequence used to amplify the 3' piece for integration into the INT1 locus.
SEQ ID NO: 5 sets out the nucleotide sequence of the GFP-dCas9-Mxi1 expression unit integrated in the genome, comprising the following elements (nucleotide positions indicated): 5' INT1 integration site (1-418); connector sequence (419-468), GFP expression cassette (473-1814); connector sequence (1819-1868); connector sequence (1869-1918); hygromycin B resistance marker cassette (1923-3735); connector sequence (3747-3796); GAL1 promoter (3797-4401); dCas9-Mxi1 (4402-8736); terminator (8737-8977); connector sequence (8798-9028); 3' INT1 integration site (9029-9362).
SEQ ID NO: 6 sets out the nucleotide sequence of the GFP-dCas9-Mxi1-T7RNAp expression unit integrated in the genome, comprising the following elements (nucleotide positions indicated): 5' INT1 integration site (1-418); connector sequence (419-468), GFP expression Cassette (473-1814); connector sequence (1819-1868); connector sequence (1869-1918); hygromycin B resistance marker Cassette (1923-3735); connector sequence (3747-3796); GAL1 promoter (3797-4401); dCas9-Mxi1 (4402-8733); T2A peptide sequence (8734-8787); T7 RNA polymerase (8788-10617); SV40 nuclear localization signal N-terminal of T7-RNAp (8791 - 8811); terminator (10618-10858); connector sequence (10859-10909); 3' INT1 integration site (10910-11243).
SEQ ID NO: 7 sets out the nucleotide sequence of the SNR52 gRNA expression vector pRN1120-AG1. The 20-nt guide-sequence (genomic target sequence) is underlined.
SEQ ID NO: 8 sets out the nucleotide sequence of the T7 gRNA expression vector pAG701. The T7 promoter sequence is shown in bold (1-18) and the 20-nt guide-sequence (genomic target sequence) is underlined (18-38). The `G' at the 3' end of the T7 promoter is transcribed and is part of the resulting gRNA. The guide RNA structural component is present at nucleotide sequences 39-118. The termination unit, consisting of a self-cleaving (self-processing) ribozyme (HDVr, 119-186) and T7 terminator (187-233), are shown in upper- and lower-case underlined italics.
SEQ ID NO: 9 sets out the nucleotide sequence of the 20-nt guide-sequence that targets the *Saccharomyces bayanus* TDH3 (SbTDH3) promoter that was used for constitutive expression of GFP in tester strains AG1 and AG2.
SEQ ID NO: 10 sets out the nucleotide sequence of a random 20-nt guide-sequence that was used for normalization.
SEQ ID NO: 11 sets out the nucleotide sequence of a strong T7 promoter that was used for gRNA expression. This sequence replaced the T7 promoter sequence that is indicated in bold in SEQ ID NO: 8 (TAATACGACTCACTATAG).
SEQ ID NO: 12 sets out the nucleotide sequence of a medium strength T7 promoter that was used for gRNA expression. This sequence replaced the T7 promoter sequence that is indicated in bold in SEQ ID NO: 8 (TAATACGACTCACTATAG).
SEQ ID NO: 13 sets out the nucleotide sequence of a weak strength T7 promoter that was used for gRNA expression. This sequence replaced the T7 promoter sequence that is indicated in bold in SEQ ID NO: 8 (TAATACGACTCACTATAG).
SEQ ID NO: 14 sets out the nucleotide sequence of CAS9 including a C-terminal SV40 nuclear localization signal codon pair optimized for expression in *Saccharomyces cerevisiae.* The sequence includes the *KI11* promoter from *Kluyveromyces lactis* and *GND2* terminator sequence from *Saccharomyces cerevisiae.*
SEQ ID NO: 15 sets out the nucleotide sequence of vector pCSN061.
SEQ ID NO: 16 sets out the nucleotide sequence of vector pRN1 120
SEQ ID NO: 17 sets out the nucleotide sequence of T7-RNA polymerase (T7 RNAP) expression unit. The sequence includes the codon pair optimized T7 RNAP gene under control of TDH3 promoter and Enol terminator sequence for expression in S. *cerevisiae*
SEQ ID NO: 18 sets out the nucleotide sequence of T7-RNApolymerase (T7 RNAP) expression unit. The sequence includes the codon pair optimized T7 RNAP gene under control of enol promoter and Enol terminator sequence for expression in S. cerevisiae
SEQ ID NO: 19 sets out the sequence of the codon pair optimized T7 RNAP gene for expression in yeast
SEQ ID NO: 20 sets out the nucleotide sequence of the *S*. *cerevisiae* tdh3 promoter (Ptdh3)
SEQ ID NO: 21 sets out the nucleotide sequence of the *S*. *cerevisiae* enol promoter (Penol)
SEQ ID NO: 22 sets out the nucleotide sequence of the S. cerevisiae enol terminator (Tenol)
SEQ ID NO: 23 sets out the nucleotide sequence of forward primer to the 5' transition of the pCSN061 backbone to the T7 RNAP expression cassette
SEQ ID NO: 24 sets out the nucleotide sequence of reverse primer to the 5' transition of the pCSN061 backbone to the T7 RNAP expression cassette
SEQ ID NO: 25 sets out the nucleotide sequence of forward primer to the 3' transition of the pCSN061 backbone to the T7 RNAP expression cassette
SEQ ID NO: 26 sets out the nucleotide sequence of reverse primer to the 3' transition of the pCSN061 backbone to the T7 RNAP expression cassette
SEQ ID NO: 27 sets out the nucleotide sequence of vector pCSN070
SEQ ID NO: 28 sets out the nucleotide sequence of vector pCSN071
SEQ ID NO: 29 sets out the nucleotide sequence of the INT1 genomic target
SEQ ID NO: 30 sets out the nucleotide sequence of the Hepatitis Delta Virus ribozyme (HDVr)
SEQ ID NO: 31 sets out the nucleotide sequence of the T7 terminator
SEQ ID NO: 32 sets out the nucleotide sequence of a weak strength T7 promoter that was used for gRNA expression
SEQ ID NO: 33 sets out the nucleotide sequence of a medium strength T7 promoter that was used for gRNA expression
SEQ ID NO: 34 sets out the nucleotide sequence of a strong T7 promoter that was used for gRNA expression
SEQ ID NO: 35 sets out the nucleotide sequence of a wild type strength T7 promoter that was used for gRNA expression
SEQ ID NO: 36 sets out the nucleotide sequence of a guide RNA expression cassette under control of the strong T7 promoter
SEQ ID NO: 37 sets out the nucleotide sequence of a guide RNA expression cassette under control of the medium T7 promoter
SEQ ID NO: 38 sets out the nucleotide sequence of a guide RNA expression cassette under control of the weak T7 promoter
SEQ ID NO: 39 sets out the nucleotide sequence of a guide RNA expression cassette under control of the wildtype T7 promoter
SEQ ID NO: 40 sets out the nucleotide sequence of the forward primer used to obtain the T7 controlled guide RNA fragment used in transformation
SEQ ID NO: 41 sets out the nucleotide sequence of the reverse primer used to obtain the T7 controlled guide RNA fragment used in transformation
SEQ ID NO: 42 sets out the nucleotide sequence of the guide RNA fragment controlled by strong T7 promoter used in transformation flanked by 84 bp sequence of pRN1120 on the 5' side and 93 bp sequence of pRN1120 on the 3' side for in vivo assembly into the vector pRN1120
SEQ ID NO: 43 sets out the nucleotide sequence of the guide RNA fragment controlled by medium T7 promoter used in transformation flanked by 84 bp sequence of pRN1120 on the 5' side and 93 bp sequence of pRN1120 on the 3' side for in vivo assembly into the vector pRN1120
SEQ ID NO: 44 sets out the nucleotide sequence of the guide RNA fragment controlled by weak T7 promoter used in transformation flanked by 84 bp sequence of pRN1120 on the 5' side and 93 bp sequence of pRN1120 on the 3' side for in vivo assembly into the vector pRN1120
SEQ ID NO: 45 sets out the nucleotide sequence of the guide RNA fragment controlled by wild type T7 promoter used in transformation flanked by 84 bp sequence of pRN1120 on the 5' side and 93 bp sequence of pRN1120 on the 3' side for in vivo assembly into the vector pRN1120
SEQ ID NO: 46 sets out the nucleotide sequence of upper strand of the 100 bp left flank
SEQ ID NO: 47 sets out the nucleotide sequence of the 100 bp left flank in the reverse orientation complementary to SEQ ID NO: 46
SEQ ID NO: 48 sets out the nucleotide sequence of the 100 bp right flank in the forward orientation
SEQ ID NO: 49 sets out the nucleotide sequence of the 100 bp right flank in the reverse orientation complementary to SEQ ID NO: 48
SEQ ID NO: 50 sets out the nucleotide sequence of the YFP donor DNA expression cassette
SEQ ID NO: 51 sets out the nucleotide sequence forward primer to obtain the 577 bp left flank for integration of the YFP donor DNA cassette
SEQ ID NO: 52 sets out the nucleotide sequence reverse primer to obtain the 577 bp left flank for integration of the YFP donor DNA cassette
SEQ ID NO: 53 sets out the nucleotide sequence of the left flank for integration of YFP donor expression cassette in INT1 locus of S. *cerevisiae*
SEQ ID NO: 54 sets out the nucleotide sequence forward primer to obtain the 581 bp right flank for integration of the YFP donor DNA expression cassette
SEQ ID NO: 55 sets out the nucleotide sequence reverse primer to obtain the 581 bp right flank for integration of the YFP donor DNA expression cassette
SEQ ID NO: 56 sets out the nucleotide sequence of the right flank for integration of YFP donor expression cassette in INT1 locus of S. *cerevisiae*
SEQ ID NO: 57 sets out the nucleotide sequence of the forward primer for amplification of the YFP expression cassette
SEQ ID NO: 58 sets out the nucleotide sequence of the reverse primer for amplification of the YFP expression cassette
SEQ ID NO: 59 sets out the nucleotide sequence of the forward primer for amplification of the YFP donor DNA expression cassette including connector 5 on the 5' side
SEQ ID NO: 60 sets out the nucleotide sequence of the reverse primer for amplification of the YFP donor DNA expression cassette including connector 3 on the 3' side
SEQ ID NO: 61 sets out the sequence of the guide RNA structural element as described by DiCarlo
SEQ ID NO: 62 sets out the nucleotide sequence of ordered gBlock donor DNA with fwnA as target
SEQ ID NO: 63 sets out the nucleotide sequence of TOPO vector with donor DNA (target fwnA) result of cloning gBIock donor DNA in TOPO-vector
SEQ ID NO: 64 sets out the forward primer sequence used to amplify the donor DNA (target fwnA)
SEQ ID NO: 65 sets out the reverse primer sequence used to amplify the donor DNA (target fwnA)
SEQ ID NO: 66 sets out the forward primer sequence to amplify the Cas9 cassette with additional Kpnl-flank for ligation in AMA-vector
SEQ ID NO: 67 sets out the reverse primer sequence to amplify the Cas9 cassette with additional Kpnl-flank for ligation in AMA-vector
SEQ ID NO: 68 sets out the nucleotide sequence of BG-AMA17 (Cas9/hygB) - result of ligation PCR-fragment (Cas9-cassette with Kpnl-flanks) and BG-AMA8
SEQ ID NO: 69 sets out the nucleotide sequence of ordered gBlock with T7 gRNA cassette - T7.pro WT sgRNA fwnA
SEQ ID NO: 70 sets out the nucleotide sequence of ordered gBlock with T7 gRNA cassette - T7.pro strong sgRNA fwnA
SEQ ID NO: 71 sets out the nucleotide sequence of BG-AMA18 (Cas9/hygB/T7 wt sgRNA fwnA) - Golden Gate product BG-AMA17 with gBlock T7.pro WT sgRNA fwnA
SEQ ID NO: 72 sets out the nucleotide sequence of BG-AMA19 (Cas9/hygB/T7 strong sgRNA fwnA) - Golden Gate product BG-AMA17 with gBlock T7.pro strong sgRNA fwnA
SEQ ID NO: 73 sets out the forward primer sequence used to check the cloned T7 gRNA cassette in AMA-vector (BG-AMA18 and BG-AMA19) by GoldenGate
SEQ ID NO: 74 sets out the reverse primer sequence used to check the cloned T7 gRNA cassette in AMA-vector (BG-AMA18 and BG-AMA19) by GoldenGate
SEQ ID NO: 75 sets out the forward primer sequence to amplify part of the fwnA gene to produce DNA fragments for sequencing and primer also used for sequencing reaction to check correct integration of door DNA in the genome
SEQ ID NO: 76 sets out the reverse primer sequence to amplify part of the fwnA gene to produce DNA fragments for sequencing

### Sequences

SEQ ID NO: 7 
CATATATTTATTAATCAAACAGTTTTAGAGCTAGAAATAGCAAGTTAAAATAAGGCTAGTCCGTTATCAACTTGAAAAAGTGGCACCG
AGTCGGTGCTTTTGTTTTTTTGTTTTTTATGTCTGGGGGGCCCGGTACCCAGCTTTTGTTCCCTTTAGTGAGGGTTAATTCCGAGCTT
GGCGTAATCATGGTCATAGCTGTTTCCTGTGTGAAATTGTTATCCGCTCACAATTCCACACAACATAGGAGCCGGAAGCATAAAGTGT
AAAGCCTGGGGTGCCTAATGAGTGAGGTAACTCACATTAATTGCGTTGCGCTCACTGCCCGCTTTCCAGTCGGGAAACCTGTCGTGCC
AGCTGCATTAATGAATCGGCCAACGCGCGGGGAGAGGCGGTTTGCGTATTGGGCGCTCTTCCGCTTCCTCGCTCACTGACTCGCTGCG
CTCGGTCGTTCGGCTGCGGCGAGCGGTATCAGCTCACTCAAAGGCGGTAATACGGTTATCCACAGAATCAGGGGATAACGCAGGAAAG
AACATGTGAGCAAAAGGCCAGCAAAAGGCCAGGAACCGTAAAAAGGCCGCGTTGCTGGCGTTTTTCCATAGGCTCGGCCCCCCTGACG
AGCATCACAAAAATCGACGCTCAAGTCAGAGGTGGCGAAACCCGACAGGACTATAAAGATACCAGGCGTTCCCCCCTGGAAGCTCCCT
CGTGCGCTCTCCTGTTCCGACCCTGCCGCTTACCGGATACCTGTCCGCCTTTCTCCCTTCGGGAAGCGTGGCGCTTTCTCAATGCTCA
CGCTGTAGGTATCTCAGTTCGGTGTAGGTCGTTCGCTCCAAGCTGGGCTGTGTGCACGAACCCCCCGTTCAGCCCGACCGCTGCGCCT
TATCCGGTAACTATCGTCTTGAGTCCAACCCGGTAAGACACGACTTATCGCCACTGGCAGCAGCCACTGGTAACAGGATTAGCAGAGC
GAGGTATGTAGGCGGTGCTACAGAGTTCTTGAAGTGGTGGCCTAACTACGGCTACACTAGAAGGACAGTATTTGGTATCTGCGCTCTG
CTGAAGCCAGTTACCTTCGGAAAAAGAGTTGGTAGCTCTTGATCCGGCAAACAAACCACCGCTGGTAGCGGTGGTTTTTTTGTTTGCA
AGCAGCAGATTACGCGCAGAAAAAAAGGATCTCAAGAAGATCCTTTGATCTTTTCTACGGGGTCTGACGCTCAGTGGAACGAAAACTC
ACGTTAAGGGATTTTGGTCATGAGATTATCAAAAAGGATCTTCACCTAGATCCTTTTAAATTAAAAATGAAGTTTTAAATCAATCTAA
AGTATATATGAGTAAACTTGGTCTGACAGTTACCAATGCTTAATCAGTGAGGCACCTATCTCAGCGATCTGTCTATTTCGTTCATCCA
TAGTTGCCTGACTGCCCGTCGTGTAGATAACTACGATACGGGAGGGCTTACCATCTGGCCCCAGTGCTGCAATGATACCGCGAGACCC
ACGCTCACCGGCTCCAGATTTATCAGCAATAAACCAGCCAGCCGGAAGGGCCGAGCGCAGAAGTGGTCCTGCAACTTTATCCGCCTCC
ATCCAGTCTATTAATTGTTGCCGGGAAGCTAGAGTAAGTAGTTCGCCAGTTAATAGTTTGCGCAACGTTGTTGCCATTGCTACAGGCA
TCGTGGTGTCACGCTCGTCGTTTGGTATGGCTTCATTCAGCTCCGGTTCCCAACGATCAAGGCGAGTTACATGATCCCCCATGTTGTG
AAAAAAAGCGGTTAGCTCCTTCGGTCCTCCGATCGTTGTCAGAAGTAAGTTGGCCGCAGTGTTATCACTCATGGTTATGGCAGCACTG
CATAATTCTCTTACTGTCATGCCATCCGTAAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAGAACTTTAAAAGTGCTCATCATTGGAAAACG
TTCTTCGGGGCGAAAACTCTCAAGGATCTTACCGCTGTTGAGATCCAGTTCGATGTAACCCACTCGTGCACCCAACTGATCTTCAGCA
TCTTTTACTTTCACCAGCGTTTCTGGGTGAGCAAAAACAGGAAGGCAAAATGCCGCAAAAAAGGGAATAAGGGCGACACGGAAATGTT
GAATACTCATACTCTTCCTTTTTCAATATTATTGAAGCATTTATCAGGGTTATTGTCTCATGAGCGGATACATATTTGAATGTATTTA
 
GAAAAATAAACAAATAGGGGTTCCGCGCACATTTCCCCGAAAAGTGCCACCTGACGTCTAAGAAACCATTATTATCATGACATTAACC
TATAAAAATAGGCGTATCACGAGGCCCTTTCGTCTCGCGCGTTTCGGTGATGACGGTGAAAACCTCTGACACATGCAGCTCCCGGAGA
CGGTCACAGCTTGTCTGTAAGCGGATGCCGGGAGCAGACAAGCCCGTCAGGGCGCGTCAGCGGGTGTTGGCGGGTGTCGGGGCTGGCT
TAACTATGCGGCATCAGAGCAGATTGTACTGAGAGTGCACCATATCGACTACGTCGTAAGGCCGTTTCTGACAGAGTAAAATTCTTGA
GGGAACTTTCACCATTATGGGAAATGGTTCAAGAAGGTATTGACTTAAACTCCATCAAATGGTCAGGTCATTGAGTGTTTTTTATTTG
TTGTATTTTTTTTTTTTTAGAGAAAATCCTCCAATATCAAATTAGGAATCGTAGTTTCATGATTTTCTGTTACACCTAACTTTTTGTG
TGGTGCCCTCCTCCTTGTCAATATTAATGTTAAAGTGCAATTCTTTTTCCTTATCACGTTGAGCCATTAGTATCAATTTGCTTACCTG
TATTCCTTTACTATCCTCCTTTTTCTCCTTCTTGATAAATGTATGTAGATTGCGTATATAGTTTCGTCTACCCTATGAACATATTCCA
TTTTGTAATTTCGTGTCGTTTCTATTATGAATTTCATTTATAAAGTTTATGTACACCTAGGATCCGTCGACACTGGATGGCGGCGTTA
GTATCGAATCGACAGCAGTATAGCGACCAGCATTCACATACGATTGACGCATGATATTACTTTCTGCGCACTTAACTTCGCATCTGGG
CAGATGATGTCGAGGCGAAAAAAAATATAAATCACGCTAACATTTGATTAAAATAGAACAACTACAATATAAAAAAACTATACAAATG
ACAAGTTCTTGAAAACAAGAATCTTTTTATTGTCAGTACTAGGGGCAGGGCATGCTCATGTAGAGCGCCTGCTCGCCGTCCGAGGCGG
TGCCGTCGTACAGGGCGGTGTCCAGGCCGCAGAGGGTGAACCCCATCCGCCGGTACGCGTGGATCGCCGGTGCGTTGACGTTGGTGAC
CTCCAGCCAGAGGTGCCCGGCGCCCCGCTCGCGGGCGAACTCCGTCGCGAGCCCCATCAACGCGCGCCCGACCCCGTGCCCCCGGTGC
TCCGGGGCGACCTCGATGTCCTCGACGGTCAGCCGGCGGTTCCAGCCGGAGTACGAGACGACCACGAAGCCCGCCAGGTCGCCGTCGT
CCCCGTACGCGACGAACGTCCGGGAGTCCGGGTCGCCGTCCTCCCCGGCGTCCGATTCGTCGTCCGATTCGTCGTCGGGGAACACCTT
GGTCAGGGGCGGGTCCACCGGCACCTCCCGCAGGGTGAAGCCGTCCCCGGTGGCGGTGACGCGGAAGACGGTGTCGGTGGTGAAGGAC
CCATCCAGTGCCTCGATGGCCTCGGCGTCCCCCGGGACACTGGTGCGGTACCGGTAAGCCGTGTCGTCAAGAGTGGTCATTTTACATG
GTTGTTTATGTTCGGATGTGATGTGAGAACTGTATCCTAGCAAGATTTTAAAAGGAAGTATATGAAAGAAGAACCTCAGTGGCAAATC
CTAACCTTTTATATTTCTCTACAGGGGCGCGGCGTGGGGACAATTCAACGCGTCTGTGAGGGGAGCGTTTCCCTGCTCGCAGGTCTGC
AGCGAGGAGCCGTAATTTTTGCTTCGCGCCGTGCGGCCATCAAAATGTATGGATGCAAATGATTATACATGGGGATGTATGGGCTAAA
TGTACGGGCGACAGTCACATCATGCCCCTGAGCTGCGCACGTCAAGACTGTCAAGGAGGGTATTCTGGGCCTCCATGTCGCTGGCCGG
GTGACCCGGCGGGGACGAGGCCTTAAGTTCGAACGTACGAGCTCCGGCATTGCGAATACCGCTTTCCACAAACATTGCTCAAAAGTAT
CTCTTTGCTATATATCTCTGTGCTATATCCCTATATAACCTACCCATCCACCTTTCGCTCCTTGAACTTGCATCTAAACTCGACCTCT
ACATTTTTTATGTTTATCTCTAGTATTACTCTTTAGACAAAAAAATTGTAGTAAGAACTATTCATAGAGTGAATCGAAAACAATACGA
AAATGTAAACATTTCCTATACGTAGTATATAGAGACAAAATAGAAGAAACCGTTCATAATTTTCTGACCAATGAAGAATCATCAACGC
TATCACTTTCTGTTCACAAAGTATGCGCAATCCACATCGGTATAGAATATAATCGGGGATGCCTTTATCTTGAAAAAATGCACCCGCA
GCTTCGCTAGTAATCAGTAAACGCGGGAAGTGGAGTCAGGCTTTTTTTATGGAAGAGAAAATAGACACCAAAGTAGCCTTCTTCTAAC
CTTAACGGACCTACAGTGCAAAAAGTTATCAAGAGACTGCATTATAGAGCGCACAAAGGAGAAAAAAAGTAATCTAAGATGCTTTGTT
AGAAAAATAGCGCTCTCGGGATGCATTTTTGTAGAACAAAAAAGAAGTATAGATTCTTTGTTGGTAAAATAGCGCTCTCGCGTTGCAT
TTCTGTTCTGTAAAAATGCAGCTCAGATTCTTTGTTTGAAAAATTAGCGCTCTCGCGTTGCATTTTTGTTTTACAAAAATGAAGCACA
GATTCTTCGTTGGTAAAATAGCGCTTTCGCGTTGCATTTCTGTTCTGTAAAAATGCAGCTCAGATTCTTTGTTTGAAAAATTAGCGCT
CTCGCGTTGCATTTTTGTTCTACAAAATGAAGCACAGATGCTTCGTTAACAAAGATATGCTATTGAAGTGCAAGATGGAAACGCAGAA
AATGAACCGGGGATGCGACGTGCAAGATTACCTATGCAATAGATGCAATAGTTTCTCCAGGAACCGAAATACATACATTGTCTTCCGT
AAAGCGCTAGACTATATATTATTATACAGGTTCAAATATACTATCTGTTTCAGGGAAAACTCCCAGGTTCGGATGTTCAAAATTCAAT
GATGGGTAACAAGTACGATCGTAAATCTGTAAAACAGTTTGTCGGATATTAGGCTGTATCTCCTCAAAGCGTATTCGAATATCATTGA
GAAGCTGCAGCGTCACATCGGATAATAATGATGGCAGCCATTGTAGAAGTGCCTTTTGCATTTCTAGTCTCTTTCTCGGTCTAGCTAG
TTTTACTACATCGCGAAGATAGAATCTTAGATCACACTGCCTTTGCTGAGCTGGATCAATAGAGTAACAAAAGAGTGGTAAGGCCTCG
TTAAAGGACAAGGACCTGAGCGGAAGTGTATCGTACAGTAGACGGAGTATACTAGGTATAGTCTATAGTCCGTGGAATTAATTCTCAT
GTTTGACAGCTTATCATCGATAATCCGGAGCTAGCATGCGGCCGCTCTAGAACTAGTGGATCCCCCGGGCTGCAGTCTTTGAAAAGAT
AATGTATGATTATGCTTTCACTCATATTTATACAGAAACTTGATGTTTTCTTTCGAGTATATACAAGGTGATTACATGTACGTTTGAA
GTACAACTCTAGATTTTGTAGTGCCCTCTTGGGCTAGCGGTAAAGGTGCGCATTTTTTCACACCCTACAATGTTCTGTTCAAAAGATT
TTGGTCAAACGCTGTAGAAGTGAAAGTTGGTGCGCATGTTTCGGCGTTCGAAACTTCTCCGCAGTGAAAGATAAATGAT
SEQ ID NO: 8

TAATACGACTCACTATAGATATATTTATTAATCAAACAGTTTTAGAGCTAGAAATAGCAAGTTAAAATAAGGCTAGTCCGTTATCAAC
TTGAAAAAGTGGCACCGAGTCGGTGCTTTTGGCCGGCATGGTCCCAGCCTCCTCGCTGGCGCCGGCTGGGCAACATGCTTCGGCATGG
CGAATGGGACaaaaaatcaaactggctcaccttcgggtgggcctttttgcgtttataGGGGGGCCCGGTACCCAGCTTTTGTTCCCTT
TAGTGAGGGTTAATTCCGAGCTTGGCGTAATCATGGTCATAGCTGTTTCCTGTGTGAAATTGTTATCCGCTCACAATTCCACACAACA
TAGGAGCCGGAAGCATAAAGTGTAAAGCCTGGGGTGCCTAATGAGTGAGGTAACTCACATTAATTGCGTTGCGCTCACTGCCCGCTTT
CCAGTCGGGAAACCTGTCGTGCCAGCTGCATTAATGAATCGGCCAACGCGCGGGGAGAGGCGGTTTGCGTATTGGGCGCTCTTCCGCT

TCCTCGCTCACTGACTCGCTGCGCTCGGTCGTTCGGCTGCGGCGAGCGGTATCAGCTCACTCAAAGGCGGTAATACGGTTATCCACAG
AATCAGGGGATAACGCAGGAAAGAACATGTGAGCAAAAGGCCAGCAAAAGGCCAGGAACCGTAAAAAGGCCGCGTTGCTGGCGTTTTT
CCATAGGCTCGGCCCCCCTGACGAGCATCACAAAAATCGACGCTCAAGTCAGAGGTGGCGAAACCCGACAGGACTATAAAGATACCAG
GCGTTCCCCCCTGGAAGCTCCCTCGTGCGCTCTCCTGTTCCGACCCTGCCGCTTACCGGATACCTGTCCGCCTTTCTCCCTTCGGGAA
GCGTGGCGCTTTCTCAATGCTCACGCTGTAGGTATCTCAGTTCGGTGTAGGTCGTTCGCTCCAAGCTGGGCTGTGTGCACGAACCCCC
CGTTCAGCCCGACCGCTGCGCCTTATCCGGTAACTATCGTCTTGAGTCCAACCCGGTAAGACACGACTTATCGCCACTGGCAGCAGCC
ACTGGTAACAGGATTAGCAGAGCGAGGTATGTAGGCGGTGCTACAGAGTTCTTGAAGTGGTGGCCTAACTACGGCTACACTAGAAGGA
CAGTATTTGGTATCTGCGCTCTGCTGAAGCCAGTTACCTTCGGAAAAAGAGTTGGTAGCTCTTGATCCGGCAAACAAACCACCGCTGG
TAGCGGTGGTTTTTTTGTTTGCAAGCAGCAGATTACGCGCAGAAAAAAAGGATCTCAAGAAGATCCTTTGATCTTTTCTACGGGGTCT
GACGCTCAGTGGAACGAAAACTCACGTTAAGGGATTTTGGTCATGAGATTATCAAAAAGGATCTTCACCTAGATCCTTTTAAATTAAA
AATGAAGTTTTAAATCAATCTAAAGTATATATGAGTAAACTTGGTCTGACAGTTACCAATGCTTAATCAGTGAGGCACCTATCTCAGC
GATCTGTCTATTTCGTTCATCCATAGTTGCCTGACTGCCCGTCGTGTAGATAACTACGATACGGGAGGGCTTACCATCTGGCCCCAGT
GCTGCAATGATACCGCGAGACCCACGCTCACCGGCTCCAGATTTATCAGCAATAAACCAGCCAGCCGGAAGGGCCGAGCGCAGAAGTG
GTCCTGCAACTTTATCCGCCTCCATCCAGTCTATTAATTGTTGCCGGGAAGCTAGAGTAAGTAGTTCGCCAGTTAATAGTTTGCGCAA
CGTTGTTGCCATTGCTACAGGCATCGTGGTGTCACGCTCGTCGTTTGGTATGGCTTCATTCAGCTCCGGTTCCCAACGATCAAGGCGA
GTTACATGATCCCCCATGTTGTGAAAAAAAGCGGTTAGCTCCTTCGGTCCTCCGATCGTTGTCAGAAGTAAGTTGGCCGCAGTGTTAT
CACTCATGGTTATGGCAGCACTGCATAATTCTCTTACTGTCATGCCATCCGTAAGATGCTTTTCTGTGACTGGTGAGTACTCAACCAA
GTCATTCTGAGAATAGTGTATGCGGCGACCGAGTTGCTCTTGCCCGGCGTCAATACGGGATAATACCGCGCCACATAGCAGAACTTTA
AAAGTGCTCATCATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGCCGCAAAAAAGGG
AATAAGGGCGACACGGAAATGTTGAATACTCATACTCTTCCTTTTTCAATATTATTGAAGCATTTATCAGGGTTATTGTCTCATGAGC
GGATACATATTTGAATGTATTTAGAAAAATAAACAAATAGGGGTTCCGCGCACATTTCCCCGAAAAGTGCCACCTGACGTCTAAGAAA
CCATTATTATCATGACATTAACCTATAAAAATAGGCGTATCACGAGGCCCTTTCGTCTCGCGCGTTTCGGTGATGACGGTGAAAACCT
CTGACACATGCAGCTCCCGGAGACGGTCACAGCTTGTCTGTAAGCGGATGCCGGGAGCAGACAAGCCCGTCAGGGCGCGTCAGCGGGT
GTTGGCGGGTGTCGGGGCTGGCTTAACTATGCGGCATCAGAGCAGATTGTACTGAGAGTGCACCATATCGACTACGTCGTAAGGCCGT
TTCTGACAGAGTAAAATTCTTGAGGGAACTTTCACCATTATGGGAAATGGTTCAAGAAGGTATTGACTTAAACTCCATCAAATGGTCA
GGTCATTGAGTGTTTTTTATTTGTTGTATTTTTTTTTTTTTAGAGAAAATCCTCCAATATCAAATTAGGAATCGTAGTTTCATGATTT
TCTGTTACACCTAACTTTTTGTGTGGTGCCCTCCTCCTTGTCAATATTAATGTTAAAGTGCAATTCTTTTTCCTTATCACGTTGAGCC
ATTAGTATCAATTTGCTTACCTGTATTCCTTTACTATCCTCCTTTTTCTCCTTCTTGATAAATGTATGTAGATTGCGTATATAGTTTC
GTCTACCCTATGAACATATTCCATTTTGTAATTTCGTGTCGTTTCTATTATGAATTTCATTTATAAAGTTTATGTACACCTAGGATCC
GTCGACACTGGATGGCGGCGTTAGTATCGAATCGACAGCAGTATAGCGACCAGCATTCACATACGATTGACGCATGATATTACTTTCT
CCGCACTTAACTTCGCATCTGGGCAGATGATGTCCAGGCGAAAAAAAATATAAATCACGCTAACATTTCATTAAAATAGAACAACTAC
AATATAAAAAAACTATACAAATGACAAGTTCTTGAAAACAAGAATCTTTTTATTGTCAGTACTAGGGGCAGGGCATGCTCATGTAGAG
CGCCTGCTCGCCGTCCGAGGCGGTGCCGTCGTACAGGGCGGTGTCCAGGCCGCAGAGGGTGAACCCCATCCGCCGGTACGCGTGGATC
GCCGGTGCGTTGACGTTGGTGACCTCCAGCCAGAGGTGCCCGGCGCCCCGCTCGCGGGCGAACTCCGTCGCGAGCCCCATCAACGCGC
GCCCGACCCCGTGCCCCCGGTGCTCCGGGGCGACCTCGATGTCCTCGACGGTCAGCCGGCGGTTCCAGCCGGAGTACGAGACGACCAC
GAAGCCCGCCAGGTCGCCGTCGTCCCCGTACGCGACGAACGTCCGGGAGTCCGGGTCGCCGTCCTCCCCGGCGTCCGATTCGTCGTCC
GATTCGTCGTCGGGGAACACCTTGGTCAGGGGCGGGTCCACCGGCACCTCCCGCAGGGTGAAGCCGTCCCCGGTGGCGGTGACGCGGA
AGACGGTGTCGGTGGTGAAGGACCCATCCAGTGCCTCGATGGCCTCGGCGTCCCCCGGGACACTGGTGCGGTACCGGTAAGCCGTGTC
GTCAAGAGTGGTCATTTTACATGGTTGTTTATGTTCGGATGTGATGTGAGAACTGTATCCTAGCAAGATTTTAAAAGGAAGTATATGA
AAGAAGAACCTCAGTGGCAAATCCTAACCTTTTATATTTCTCTACAGGGGCGCGGCGTGGGGACAATTCAACGCGTCTGTGAGGGGAG
CGTTTCCCTGCTCGCAGGTCTGCAGCGAGGAGCCGTAATTTTTGCTTCGCGCCGTGCGGCCATCAAAATGTATGGATGCAAATGATTA
TACATGGGGATGTATGGGCTAAATGTACGGGCGACAGTCACATCATGCCCCTGAGCTGCGCACGTCAAGACTGTCAAGGAGGGTATTC
TGGGCCTCCATGTCGCTGGCCGGGTGACCCGGCGGGGACGAGGCCTTAAGTTCGAACGTACGAGCTCCGGCATTGCGAATACCGCTTT
CCACAAACATTGCTCAAAAGTATCTCTTTGCTATATATCTCTGTGCTATATCCCTATATAACCTACCCATCCACCTTTCGCTCCTTGA
ACTTGCATCTAAACTCGACCTCTACATTTTTTATGTTTATCTCTAGTATTACTCTTTAGACAAAAAAATTGTAGTAAGAACTATTCAT
AGAGTGAATCGAAAACAATACGAAAATGTAAACATTTCCTATACGTAGTATATAGAGACAAAATAGAAGAAACCGTTCATAATTTTCT
GACCAATGAAGAATCATCAACGCTATCACTTTCTGTTCACAAAGTATGCGCAATCCACATCGGTATAGAATATAATCGGGGATGCCTT
TATCTTGAAAAAATGCACCCGCAGCTTCGCTAGTAATCACTAAACGCGGGAAGTGGAGTCAGGCTTTTTTTATGGAACACAAAATAGA
CACCAAAGTAGCCTTCTTCTAACCTTAACGGACCTACAGTGCAAAAAGTTATCAAGAGACTGCATTATAGAGCGCACAAAGGAGAAAA
AAAGTAATCTAAGATGCTTTGTTAGAAAAATAGCGCTCTCGGGATGCATTTTTGTAGAACAAAAAAGAAGTATAGATTCTTTGTTGGT

AAAATAGCGCTCTCGCGTTGCATTTCTGTTCTGTAAAAATGCAGCTCAGATTCTTTGTTTGAAAAATTAGCGCTCTCGCGTTGCATTT
TTGTTTTACAAAAATGAAGCACAGATTCTTCGTTGGTAAAATAGCGCTTTCGCGTTGCATTTCTGTTCTGTAAAAATGCAGCTCAGAT
TCTTTGTTTGAAAAATTAGCGCTCTCGCGTTGCATTTTTGTTCTACAAAATGAAGCACAGATGCTTCGTTAACAAAGATATGCTATTG
AAGTGCAAGATGGAAACGCAGAAAATGAACCGGGGATGCGACGTGCAAGATTACCTATGCAATAGATGCAATAGTTTCTCCAGGAACC
GAAATACATACATTGTCTTCCGTAAAGCGCTAGACTATATATTATTATACAGGTTCAAATATACTATCTGTTTCAGGGAAAACTCCCA
GGTTCGGATGTTCAAAATTCAATGATGGGTAACAAGTACGATCGTAAATCTGTAAAACAGTTTGTCGGATATTAGGCTGTATCTCCTC
AAAGCGTATTCGAATATCATTGAGAAGCTGCAGCGTCACATCGGATAATAATGATGGCAGCCATTGTAGAAGTGCCTTTTGCATTTCT
AGTCTCTTTCTCGGTCTAGCTAGTTTTACTACATCGCGAAGATAGAATCTTAGATCACACTGCCTTTGCTGAGCTGGATCAATAGAGT
AACAAAAGAGTGGTAAGGCCTCGTTAAAGGACAAGGACCTGAGCGGAAGTGTATCGTACAGTAGACGGAGTATACTAGGTATAGTCTA
TAGTCCGTGGAATTAATTCTCATGTTTGACAGCTTATCATCGATAATCCGGAGCTAGCATGCGGCCGCTCTAGAACTAGTGGATCCCC
CGGGCTGCAG

### Detailed description

The invention relates to the use of a single-subunit DNA-dependent RNA polymerase selected from a T3, SP6, K11 or T7 RNA polymerase, for the expression within a fungal cell of a guide-RNA for an RNA-guided nuclease system, wherein the guide-RNA is encoded by a polynucleotide that is operably linked to a viral single-subunit DNA-dependent RNA polymerase promoter selected from a T3, SP6, K11 or T7 RNA polymerase promoter and wherein the fungal cell is a yeast cell or a filamentous fungus cell.

The use, the single-subunit DNA-dependent RNA polymerase, the viral single-subunit DNA-dependent RNA polymerase, the T3, SP6, K11 and T7 RNA polymerase, the guide-RNA, the RNA-guided nuclease system, the single-subunit DNA-dependent RNA polymerase promoter, viral single-subunit DNA-dependent RNA polymerase promoter and the T3, SP6, K11 or T7 RNA polymerase promoter are herein referred to as the single-subunit DNA-dependent RNA polymerase, the viral single-subunit DNA-dependent RNA polymerase, the T3, SP6, K11 and T7 RNA polymerase, the guide-RNA, the RNA-guided nuclease system, the single-subunit DNA-dependent RNA polymerase promoter, viral single-subunit DNA-dependent RNA polymerase promoter and the T3, SP6, K11 or T7 RNA polymerase promoter according to the invention. The guide-RNA according to the invention is preferably an uncapped RNA; i.e. it does not have a 5'-cap (see general definitions).

For the sake of completeness, "a" is defined elsewhere herein as "at least one"; e.g. a single-subunit DNA-dependent RNA polymerase is thus to be construed as one, two, three or more single-subunit DNA-dependent RNA polymerase. The guide-RNA is preferably a guide-RNA of pre-determined sequence; as such any process of nature is excluded and only engineered (man-made) processes and products are contemplated to be within the scope of the present invention.

A single-subunit DNA-dependent RNA polymerase according to the invention is an RNA polymerase that transcribes from a DNA template and is a member of the viral single-subunit RNA polymerases. Such RNA polymerases are known to the person skilled in the art. The viral single-subunit DNA-dependent RNA polymerase is selected from the group of T3, SP6, K11 and T7 RNA polymerase. The cell according to the invention may bel a yeast cell or a filamentous fungal cell; all as defined in the general definitions herein.

The term "expression" is known to the person skilled in the art and is in the context of the invention herein defined as the process by which a polynucleotide is transcribed from a polynucleotide template (e.g. a DNA template polynucleotide is transcribed into an mRNA polynucleotide transcript or other RNA transcript) and/or the process by which an mRNA transcript is subsequently translated into peptides, polypeptides, or proteins. Transcripts and encoded polypeptides may be collectively referred to as "gene product". If the polynucleotide transcript is derived from a genomic template DNA, expression may include splicing of the mRNA transcript in a host cell. The term "modulating expression" refers herein to increased or reduced expression compared to a parent host cell wherein expressing is not modulated when assayed using the same conditions. Reduced expression may be a reduced amount of transcript such as mRNA and/or a reduced amount of translation product such as a polypeptide. It follows that increased expression may be an enhanced amount of transcript such as mRNA and/or an enhanced amount of translation product such as a polypeptide. For the sake of clarity, expression of a guide-RNA means that a guide-RNA according to the invention is produced from a DNA template by a single-subunit DNA-dependent RNA polymerase according to the invention.

A guide-RNA (also referred to as gRNA or as sgRNA) is a guide-polynucleotide comprised of ribonucleotides and comprises at least a guide-sequence that is able to hybridize with a target-polynucleotide and is able to direct sequence-specific binding of the RNA-guided nuclease system to a target-polynucleotide. The person skilled in the art is familiar with guide-RNA's, see e.g. Qi et al., 2013 and Tycko et al, 2016. In the use according to the invention, the promoter and RNA polymerase are a compatible set. In other words, expression of the guide-RNA according to the invention is driven by a promoter according to the invention that is able to initiate transcription of the guide-RNA from its encoding DNA template and said transcription is performed by an RNA polymerase according to the invention that recognizes the promoter to initiate expression from. As an example, expression of a guide-RNA according to the invention may be driven by a T7 promoter and transcription be performed by a T7 RNA polymerase.

In the use according to the invention, the RNA polymerase according to the invention may be either brought into the cell as a polypeptide in its active from or as a pro-peptide that is activated after delivery into the cell. The RNA polymerase according to the invention may also be expressed within the cell from a polynucleotide, such as an expression construct. Such expression construct may be any type of expression construct such as a linear nucleic acid construct, a genome or a vector, preferably a plasmid. A preferred nucleic acid construct, vector of plasmid comprises a selectable marker. A selectable marker is a product of a polynucleotide of interest which product provides for biocide or viral resistance, resistance to heavy metals, prototrophy to auxotrophs, and the like. Selectable markers include, but are not limited to, amdS (acetamidase), argB (ornithinecarbamoyltransferase), bar (phosphinothricinacetyltransferase), hygB (hygromycin phosphotransferase), niaD (nitrate reductase), pyrG (orotidine-5'-phosphate decarboxylase), sC (sulfate adenyltransferase), trpC (anthranilate synthase), ble (phleomycin resistance protein), hyg (hygromycin), NAT or NTC (nourseothricin) as well as equivalents thereof.

In the use according to the invention, the guide-RNA is encoded by a (DNA) polynucleotide that is operably linked to a single-subunit DNA-dependent RNA polymerase promoter according to the invention. Said construct comprising the polynucleotide encoding the guide-RNA and the promoter according to the invention from which the guide-RNA is expressed may be a linear nucleic acid construct, a genome or a vector, preferably said vector is a plasmid, preferably comprising a selectable marker. A linear nucleic acid may also be a plasmid that has been linearized.

The RNA-guided nuclease system according to the invention may be any RNA-guided nuclease system known to the person skilled in the art. RNA-guided nuclease systems are *inter alia* reviewed in Hsu et al, 2014; Sander and Joung, 2014; and Tycko et al, 2016 .... A well-known RNA-guided nuclease system that is preferred in the use according the invention is based on Clustered regularly interspaced short palindromic repeats (CRISPR), such as CRISPR/Cas and CRISPR/Cpf1. CRISPR/Cas variants are known to the person skilled in the art (see e.g. Nelson et al., 2016). The person skilled in the art will comprehend that an RNA-guided nuclease system according to the invention may conveniently be used for genome editing, e.g. to insert, delete and/or mutate a sequence in the genome of a cell, preferably using an exogenous polynucleotide such as a donor DNA molecule. The present invention thus also provides for an exogenous polynucleotide, that upon cleavage of the target-polynucleotide by RNA-guided nuclease system recombines with the target-polynucleotide, resulting in a modified target-polynucleotide. Such exogenous polynucleotide may be single-stranded or double-stranded. Herein, a donor DNA molecule may also be referred to as an exogenous nucleic acid molecule, a repair template or an exogenous polynucleotide. The term "exogenous" is herein to be construed as that an exogenous nucleic acid molecule is as such not natively present in the cell; the exogenous nucleic acid molecule is brought from outside into the cell. The exogenous nucleic acid molecule will mostly be foreign to the cell. However in certain embodiments, the exogenous nucleic acid molecule may be native to the cell but has been engineered outside the cell and is brought into the cell; in such case, the exogenous nucleic acid molecule may be considered native to the cell.

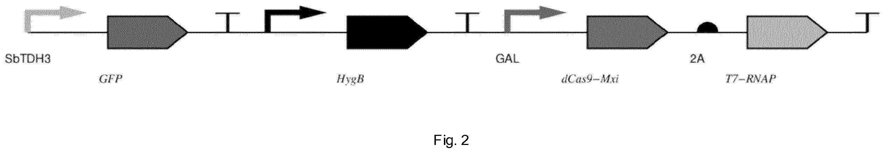

In the use according to the invention, the RNA polymerase, when expressed in the cell, is expressed from an expression construct whereon the polynucleotide encoding the RNA polymerase is operably linked to a promoter. Such promoter is known to the person skilled in the art and may be any suitable promoter, such as an inducible promoter and a constitutive promoter. An inducible promoter enables transient expression of the RNA polymerase according to the invention and/or modulation of the expression level of the RNA polymerase. The RNA polymerase may also be expressed together with another protein, such as e.g. a Cas protein, from a single promoter. In such case, the two encoding sequences may be separated by a sequence that allows expression of multiple proteins, such as a viral 2A sequence, e.g. the viral T2A sequence. Figure 2 depicts an example of such construct.

The codon usage of the RNA polymerase according to the invention may be adapted to be more compatible with the codon usage of a specific host cell. Accordingly, in the use according to the invention, the RNA polymerase is preferably a codon optimized RNA polymerase, preferably a codon pair-optimized RNA polymerase. In the alternative or in combination with a codon optimized RNA polymerase, the RNA polymerase may be a variant RNA polymerase, such as a split RNA polymerase. Such split RNA polymerase is known to the person skilled in the art and comprises several (such as one, two, three, or even four) domains that can be expressed separately and only when all expressed can aggregate to form a functional RNA polymerase. Variant single-subunit DNA-dependent RNA polymerases, such as variant T3 and T7 polymerases are known to the person skilled in the art and are *inter alia* described in US2013224793, US2015024435, US5102802, US5869320, Shis et al, 2014 Imburgio et al, 2000, Temme et al, 2012; all herein incorporated by reference).

In an embodiment, the RNA polymerase according to the invention comprises a nuclear localization signal (NLS, also referred to as nuclear targeting signal, see e.g. Benton et al, 1989), preferably either at the C- or N- terminus of the RNA polymerase, preferably at the N-terminus of the RNA polymerase. A preferred NLS is the SV40 NLS and is preferably present at the N-terminus of the RNA polymerase.

It is within the scope of the invention that more than one guide-RNA's are expressed in the cell. If multiple, distinct guide-RNA's are expressed, they can be expressed from either a sole single-subunit DNA-dependent RNA polymerase promoter or from multiple single-subunit DNA-dependent RNA polymerase promoters. Accordingly, two or more guide-RNA's are expressed from an operon-like structure driven by a sole single-subunit DNA-dependent RNA polymerase promoter, or two or more guide-RNA's are each expressed from a single-subunit DNA-dependent RNA polymerase promoter; the multiple promoters by even be distinct promoters.

In an embodiment, the guide-RNA is expressed from one or more single-subunit DNA-dependent RNA polymerase promoters from a library of single-subunit DNA-dependent RNA polymerase promoters.

In the use according to the invention, the single-subunit DNA-dependent RNA polymerase promoter may be any suitable single-subunit DNA-dependent RNA polymerase promoter known to the person skilled in the art. Such promoter may be a variant promoter, such as a chimeric promoter. A variant promoter is a promoter that has a difference in sequence as compared to the wild-type promoter found in nature while still retaining promoter activity. Variant promoters are described by Jones et al, 2015, Temme et al, 2012, Imburgio et al, 2000). A chimeric or hybrid promoter is a promoter that comprises at least two parts of distinct promoters while retaining promoter activity. Such variant promoters are *inter alia* described in Romanienko et al, 2016 and US5,017,488, which are herein incorporated by reference. In all embodiments of the invention, preferred variant T7 promoters are promoters with the sequence as set forward in SEQ ID NO's: 11, 12 and 13.

The person skilled in the art knows that several control sequences are required for proper expression of a coding sequence. Specifically, in the use according to the invention, the guide-RNA is preferably encoded by a polynucleotide that is operably linked to a single-subunit DNA-dependent RNA polymerase promoter according to the invention and to a self-processing ribozyme and/or a single-subunit DNA-dependent RNA polymerase terminator. Such self-processing ribozyme and a single-subunit DNA-dependent RNA polymerase terminator are known to the person skilled in the art. A preferred construct wherein the guide-RNA is encoded by a polynucleotide that is operably linked to a single-subunit DNA-dependent RNA polymerase promoter according to the invention and to a self-processing ribozyme and a single-subunit DNA-dependent RNA polymerase terminator is presented in the examples herein.

It is within the scope of the invention that if the guide-RNA is encoded by a polynucleotide that is operably linked to a single-subunit DNA-dependent RNA polymerase promoter and the polynucleotide and single-subunit DNA-dependent RNA polymerase promoter are present on a plasmid, that the plasmid, is assembled within the cell by integration of a single-stranded or double-stranded oligonucleotide comprising the target sequence of the guide-polynucleotide, into the plasmid. This is extensively described in EP16181781.2 and greatly facilitates expression of the guide-RNA since one or more cloning steps can be obviated. EP16181781.2 is herein incorporated by reference. In addition or in combination with the previous, the entire guide-RNA coding sequence may be provided as a double stranded oligonucleotide or as two single-stranded, complementary, oligonucleotides and as such be assembled into the expression construct, which is preferably present on a plasmid, within the cell.

In the use according to the invention, the cell is preferably deficient in an NHEJ (non-homologous end joining) component. Said component associated with NHEJ is preferably a homologue or orthologue of Ku70, Ku80, MRE11, RAD50, RAD51, RAD52, XRS2, SIR4, and/or LIG4. Alternatively, in the cell according to the invention NHEJ may be rendered deficient by use of a compound that inhibits RNA ligase IV, such as SCR7. The persons killed in the art knows how to modulate NHEJ and its effect on RNA-guided nuclease systems, see e.g. WO2014130955A1; Chu et al., 2015; Maruyama et al., 2015; Song et al., 2015 and Yu et al., 2015; all are herein incorporated by reference. Deficiency is defined herein below.

It is within the scope of the invention that the heterologous genome editing enzyme that is part of the RNA-guided nuclease system, is either present in the cell as a protein and as such may be introduced into the cell, or that the heterologous genome editing enzyme is expressed within the cell from a coding sequence. Accordingly, in the use according to the invention, the cell expresses a functional heterologous genome editing enzyme, preferably a Cas enzyme, preferably Cas9, Cas9 nickase or dCas9, or in the cell a heterologous genome editing enzyme, preferably a Cas enzyme, preferably Cas9, Cas9 nickase or dCas9, is present.

Depending on the specific rationale, the person skilled in the art has a variety of Cas enzymes at its disposal, such as the well-known wild-type Cas9 and Cpf1 (Zetsche et al, 2015), but also a Cas9 nickase or a dCas9 (reviewed by Sander and Young).

Cpf1 mediates robust DNA interference with features distinct from Cas9. Cpf1 is a single RNA-guided endonuclease lacking tracrRNA, and it utilizes a T-rich protospacer-adjacent motif. Cpf1 cleaves DNA via a staggered DNA double-stranded break. Two candidate Cpf1 enzymes from *Acidaminococcus* and *Lachnospiraceae,* with efficient genome-editing activity in human cells, have been identified.

Wildtype Cas9 nuclease creates double-strand breaks at DNA target sites with complementarity to the 5' end of a gRNA. Cas9 variants that cut one strand rather than both strands of the target DNA site are known as nickases. Cas9 contains RuvC and HNH nuclease domains. Cas9 nickase created by mutation of the RuvC nuclease domain with a D10A mutation, cleaves only the DNA strand that is complementary to and recognized by the gRNA. Cas9 nickase created by mutation of the HNH nuclease domain with a H840A mutation, cleaves only the DNA strand that does not interact with the gRNA. To improve Cas9 specificity, two D10A Cas9 nickases can be paired and are directed by a pair of appropriately oriented gRNAs. This leads to induction of two nicks that, if introduced simultaneously, would be expected to generate a 5' overhang. Catalytically inactive or `dead' Cas9 (dCas9) (e.g., with mutations in both the RuvC and HNH domains). This can be recruited by a gRNA without cleaving the target DNA site. Catalytically inactive dCas9 can be fused to a heterologous effector domain, e.g. to allow activation or repression of gene expression, as review by Didovyk *et al.,* 2016.

In the embodiments of the invention, at least several components as defined herein will at some stage be present in a cell according to the invention, such as the guide-RNA encoding polynucleotide according to the invention, the RNA-polymerase according to the invention. These components can be introduced into the cell simultaneously, or consecutively. The same applies for other components such as the components of the RNA-guided nuclease system. The person skilled in the art is aware of this and knows set-up such system.

The present invention can conveniently be used for the expression of a guide-RNA within a cell. Accordingly, in a second aspect the present invention provides for a method for expression within a fungal cell of a guide-RNA for an RNA-guided nuclease system, wherein the guide-RNA is encoded by a polynucleotide that is operably linked to a viral single-subunit DNA-dependent RNA polymerase promoter selected from a T3, SP6, K11 or T7 RNA polymerase promoter, wherein transcription of the guide-RNA is performed by a viral single-subunit DNA-dependent RNA polymerase selected from aT3, SP6, K11 or T7 RNA polymerase, and wherein the fungal cell is a yeast cell or a filamentous fungus cell. Said method is herein referred to as a method according to the invention. All features in this aspect of the invention are preferably the corresponding features defined in the first aspect of the invention.

Preferably, in a method according to the invention, the RNA polymerase is expressed within the cell from a linear nucleic acid construct, from a genome or from a vector, preferably a plasmid. A preferred nucleic acid construct, vector of plasmid comprises a selectable marker. A selectable marker is a product of a polynucleotide of interest which product provides for biocide or viral resistance, resistance to heavy metals, prototrophy to auxotrophs, and the like. Selectable markers include, but are not limited to, amdS (acetamidase), argB (ornithinecarbamoyltransferase), bar (phosphinothricinacetyltransferase), hygB (hygromycin phosphotransferase), niaD (nitrate reductase), pyrG (orotidine-5'-phosphate decarboxylase), sC (sulfate adenyltransferase), trpC (anthranilate synthase), ble (phleomycin resistance protein), hyg (hygromycin), NAT or NTC (Nourseothricin) as well as equivalents thereof.

Preferably, in a method according to the invention, the guide-RNA is expressed from a linear nucleic acid construct, from a genome or from a vector, preferably a plasmid, preferably comprising a selectable marker a defined elsewhere herein.

Preferably, in a method according to the invention, the RNA-guided nuclease system is based on CRISPR, such as CRISPR/Cas and CRISPR/Cpf1 as defined elsewhere herein.

The cell according to the invention may be a yeast cell or a filamentous fungal cell..

Preferably, in a method according to the invention, the RNA polymerase is expressed from an inducible promoter as defined elsewhere herein.

Preferably, in a method according to the invention, the RNA polymerase is a codon optimized RNA polymerase as defined elsewhere herein and/or a split RNA polymerase as defined elsewhere herein.

Preferably, in a method according to the invention, the RNA polymerase has a nuclear localization signal (NLS) at the C- or N- terminus, more preferably a SV40 NLS at the N-terminus of the RNA polymerase; all as defined elsewhere herein.

Preferably, in a method according to the invention, multiple, distinct guide-RNA's are expressed from either a sole single-subunit DNA-dependent RNA polymerase promoter or from multiple single-subunit DNA-dependent RNA polymerase promoters; all as defined elsewhere herein. Preferably, in a method according to the invention, the guide-RNA is expressed from one or more single-subunit DNA-dependent RNA polymerase promoters from a library of single-subunit DNA-dependent RNA polymerase promoters; all as defined elsewhere herein.

Preferably, in a method according to the invention, the single-subunit DNA-dependent RNA polymerase promoter is a variant single-subunit DNA-dependent RNA polymerase promoter, such as a chimeric promoter; all as defined elsewhere herein.

Preferably, in a method according to the invention, the guide-RNA is encoded by a polynucleotide that is operably linked to a single-subunit DNA-dependent RNA polymerase promoter and to a self-processing ribozyme and/or a single-subunit DNA-dependent RNA polymerase terminator; all as defined elsewhere herein.

Preferably, in a method according to the invention, the guide-RNA is encoded by a polynucleotide that is operably linked to a single-subunit DNA-dependent RNA polymerase promoter, wherein the polynucleotide and single-subunit DNA-dependent RNA polymerase promoter are present on a plasmid, and wherein the plasmid, is assembled within the cell by integration of a single-stranded or double-stranded oligonucleotide comprising the target sequence of the guide-polynucleotide, into the plasmid; all as defined elsewhere herein.

Preferably, in a method according to the invention, the cell is deficient in an NHEJ (non-homologous end joining) component. Said component associated with NHEJ is preferably a homologue or orthologue of the yeast Ku70, Ku80, MRE11, RAD50, RAD51, RAD52, XRS2, SIR4, and/or LIG4. Alternatively, in the cell according to the invention NHEJ may be rendered deficient by use of a compound that inhibits RNA ligase IV, such as SCR7. Deficiency is defined elsewhere herein.

Preferably, in a method according to the invention, the cell expresses a functional heterologous genome editing enzyme, preferably a Cas enzyme, preferably Cas9, Cas9 nickase or dCas9, or wherein in the cell a heterologous genome editing enzyme, preferably a Cas enzyme, preferably Cas9, Cas9 nickase or dCas9, is present; all as defined elsewhere herein.

In a third aspect the invention provides for a composition comprising the fungal cell, the RNA polymerase and the guide-RNA encoding polynucleotide operably linked to the promoter as defined in the first aspect of the invention. Said composition is herein referred to as a composition according to the invention.

Disclosed herein is a cell obtainable or a cell obtained by a method according to second aspect of the invention.

Further, there is provided a yeast cell or a filamentous fungal cell comprising at least the RNA polymerase and the guide-RNA encoding polynucleotide operably linked to the promoter; all as defined in the first aspect of the invention. Preferably, said cell is capable of producing a compound of interest. Preferably, said cell further comprises an RNA-guided nuclease system as defined previously herein.

Further provided is a method for the production of a compound of interest comprising culturing said yeast cell or filamentous fungal cell capable of producing a compound of interest under conditions conducive to the production of the compound of interest and, optionally, isolating or purifying said compound of interest.

A compound of interest in the context of all embodiments of the invention may be any biological compound. The biological compound may be biomass or a biopolymer or a metabolite. The biological compound may be encoded by a single polynucleotide or a series of polynucleotides composing a biosynthetic or metabolic pathway or may be the direct result of the product of a single polynucleotide or products of a series of polynucleotides, the polynucleotide may be a gene, the series of polynucleotide may be a gene cluster. In all embodiments of the present invention, the single polynucleotide or series of polynucleotides encoding the biological compound of interest or the biosynthetic or metabolic pathway associated with the biological compound of interest, are preferred targets for the compositions and methods according to the present invention. The biological compound may be native to the host cell or heterologous to the host cell.

The term "heterologous biological compound" is defined herein as a biological compound which is not native to the cell; or a native biological compound in which structural modifications have been made to alter the native biological compound.

The term "biopolymer" is defined herein as a chain (or polymer) of identical, similar, or dissimilar subunits (monomers). The biopolymer may be any biopolymer. The biopolymer may for example be, but is not limited to, a nucleic acid, polyamine, polyol, polypeptide (or polyamide), or polysaccharide.

The biopolymer may be a polypeptide. The polypeptide may be any polypeptide having a biological activity of interest. The term "polypeptide" is not meant herein to refer to a specific length of the encoded product and, therefore, encompasses peptides, oligopeptides, and proteins. The term polypeptide refers to polymers of amino acids of any length. The polymer may he linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics. Polypeptides further include naturally occurring allelic and engineered variations of the above- mentioned polypeptides and hybrid polypeptides. The polypeptide may be native or may be heterologous to the host cell. The polypeptide may be a collagen or gelatine, or a variant or hybrid thereof. The polypeptide may be an antibody or parts thereof, an antigen, a clotting factor, an enzyme, a hormone or a hormone variant, a receptor or parts thereof, a regulatory protein, a structural protein, a reporter, or a transport protein, protein involved in secretion process, protein involved in folding process, chaperone, peptide amino acid transporter, glycosylation factor, transcription factor, synthetic peptide or oligopeptide, intracellular protein. The intracellular protein may be an enzyme such as, a protease, ceramidases, epoxide hydrolase, aminopeptidase, acylases, aldolase, hydroxylase, aminopeptidase, lipase. The polypeptide may also be an enzyme secreted extracellularly. Such enzymes may belong to the groups of oxidoreductase, transferase, hydrolase, lyase, isomerase, ligase, catalase, cellulase, chitinase, cutinase, deoxyribonuclease, dextranase, esterase. The enzyme may be a carbohydrase, e.g. cellulases such as endoglucanases, β-glucanases, cellobiohydrolases or β-glucosidases, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, or amylolytic enzymes; hydrolase, isomerase, or ligase, phosphatases such as phytases, esterases such as lipases, proteolytic enzymes, oxidoreductases such as oxidases, transferases, or isomerases. The enzyme may be a phytase. The enzyme may be an aminopeptidase, asparaginase, amylase, a maltogenic amylase, carbohydrase, carboxypeptidase, endo-protease, metallo-protease, serine-protease catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, galactolipase, chlorophyllase, polyphenoloxidase, ribonuclease, transglutaminase, or glucose oxidase, hexose oxidase, monooxygenase.

According to the invention, a compound of interest can be a polypeptide or enzyme with improved secretion features as described in WO2010/102982. According to the present invention, a compound of interest can be a fused or hybrid polypeptide to which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide.

Techniques for producing fusion polypeptides are known in the art, and include, ligating the coding sequences encoding the polypeptides so that they are in frame and expression of the fused polypeptide is under control of the same promoter(s) and terminator. The hybrid polypeptides may comprise a combination of partial or complete polypeptide sequences obtained from at least two different polypeptides wherein one or more may be heterologous to the host cell. Example of fusion polypeptides and signal sequence fusions are for example as described in WO2010/121933.

The biopolymer may be a polysaccharide. The polysaccharide may be any polysaccharide, including, but not limited to, a mucopolysaccharide (e. g., heparin and hyaluronic acid) and nitrogen-containing polysaccharide (e.g., chitin). In a preferred option, the polysaccharide is hyaluronic acid.

A polynucleotide coding for the compound of interest or coding for a compound involved in the production of the compound of interest according to the invention may encode an enzyme involved in the synthesis of a primary or secondary metabolite, such as organic acids, carotenoids, (beta-lactam) antibiotics, and vitamins. Such metabolite may be considered as a biological compound according to the present invention.

The term "metabolite" encompasses both primary and secondary metabolites; the metabolite may be any metabolite. Preferred metabolites are citric acid, gluconic acid, adipic acid, fumaric acid, itaconic acid and succinic acid.

A metabolite may be encoded by one or more genes, such as in a biosynthetic or metabolic pathway. Primary metabolites are products of primary or general metabolism of a cell, which are concerned with energy metabolism, growth, and structure. Secondary metabolites are products of secondary metabolism (see, for example, R. B. Herbert, The Biosynthesis of Secondary Metabolites, Chapman and Hall, New York, 1981).

A primary metabolite may be, but is not limited to, an amino acid, fatty acid, nucleoside, nucleotide, sugar, triglyceride, or vitamin.

A secondary metabolite may be, but is not limited to, an alkaloid, coumarin, flavonoid, polyketide, quinine, steroid, peptide, or terpene. The secondary metabolite may be an antibiotic, antifeedant, attractant, bacteriocide, fungicide, hormone, insecticide, or rodenticide. Preferred antibiotics are cephalosporins and beta-lactams. Other preferred metabolites are exo-metabolites. Examples of exo-metabolites are Aurasperone B, Funalenone, Kotanin, Nigragillin, Orlandin, other naphtho-γ-pyrones, Pyranonigrin A, Tensidol B, Fumonisin B2 and Ochratoxin A.

The biological compound may also be the product of a selectable marker. A selectable marker is a product of a polynucleotide of interest which product provides for biocide or viral resistance, resistance to heavy metals, prototrophy to auxotrophs, and the like. Selectable markers include, but are not limited to, amdS (acetamidase), argB (ornithinecarbamoyltransferase), bar (phosphinothricinacetyltransferase), hygB (hygromycin phosphotransferase), niaD (nitrate reductase), pyrG (orotidine-5'-phosphate decarboxylase), sC (sulfate adenyltransferase), trpC (anthranilate synthase), ble (phleomycin resistance protein), hyg (hygromycin), NAT or NTC (Nourseothricin) as well as equivalents thereof.

According to the invention, a compound of interest is preferably a polypeptide as described in the list of compounds of interest.

According to another embodiment of the invention, a compound of interest is preferably a metabolite.

The yeast cell or filamentous fungal cell according to the invention may already be capable of producing a compound of interest. A yeast cell or filamentous fungal cell according to the invention may also be provided with a homologous or heterologous nucleic acid construct that encodes a polypeptide wherein the polypeptide may be the compound of interest or a polypeptide involved in the production of the compound of interest. The person skilled in the art knows how to modify a microbial host cell such that it is capable of producing a compound of interest.

### General definitions

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The terms "a" and "an" are used herein to refer to one or to more than one (i.e. to one or at least one) of the grammatical object of the article. By way of example, "an element" may mean one element or more than one element.

The word "about" or "approximately" when used in association with a numerical value (e.g. about 10) preferably means that the value may be the given value (of 10) more or less 1% of the value. CRISPR interference (CRISPRi) is a genetic perturbation technique that allows for sequence-specific repression or activation of gene expression in prokaryotic and eukaryotic cells.

A polynucleotide refers herein to a polymeric form of nucleotides of any length or a defined specific length-range or length, of either deoxyribonucleotides or ribonucleotides, or mixes or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA (tRNA), ribosomal RNA (rRNA), short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, oligonucleotides and primers. A polynucleotide may comprise natural and non-natural nucleotides and may comprise one or more modified nucleotides, such as a methylated nucleotide and a nucleotide analogue or nucleotide equivalent wherein a nucleotide analogue or equivalent is defined as a residue having a modified base, and/or a modified backbone, and/or a non-natural internucleoside linkage, or a combination of these modifications. As desired, modifications to the nucleotide structure may be introduced before or after assembly of the polynucleotide. A polynucleotide may be further modified after polymerization, such as by conjugation with a labeling compound.

In general, codon optimization refers to a process of modifying a nucleic acid sequence for enhanced expression in a host cell of interest by replacing at least one codon (e.g. more than 1, 2, 3, 4, 5, 10, 15, 20, 25, 50, or more codons) of a native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database", and these tables can be adapted in a number of ways. See e.g. Nakamura, Y., et al., 2000. Computer algorithms for codon optimizing a particular sequence for expression in a particular host cell are also available, such as Gene Forge (Aptagen; Jacobus, PA), are also available. Preferably, one or more codons (e.g. 1, 2, 3, 4, 5, 10, 15, 20, 25, 50, or more, or all codons) in a sequence encoding a Cas protein correspond to the most frequently used codon for a particular amino acid. Preferred methods for codon optimization are described in WO2006/077258 and WO2008/000632). WO2008/000632 addresses codon-pair optimization. Codon-pair optimization is a method wherein the nucleotide sequences encoding a polypeptide have been modified with respect to their codon-usage, in particular the codon-pairs that are used, to obtain improved expression of the nucleotide sequence encoding the polypeptide and/or improved production of the encoded polypeptide. Codon pairs are defined as a set of two subsequent triplets (codons) in a coding sequence. The amount of Cas protein in a source in a composition according to the present invention may vary and may be optimized for optimal performance.

In an RNA molecule with a 5'-cap, a 7-methylguanylate residue is located on the 5' terminus of the RNA (such as typically in mRNA in eukaryotes). RNA polymerase II (Pol II) transcribes mRNA in eukaryotes. Messenger RNA capping occurs generally as follows: The most terminal 5' phosphate group of the mRNA transcript is removed by RNA terminal phosphatase, leaving two terminal phosphates. A guanosine monophosphate (GMP) is added to the terminal phosphate of the transcript by a guanylyl transferase, leaving a 5'-5' triphosphate-linked guanine at the transcript terminus. Finally, the 7-nitrogen of this terminal guanine is methylated by a methyl transferase. The terminology "not having a 5'-cap" herein is used to refer to RNA having, for example, a 5'-hydroxyl group instead of a 5'-cap. Such RNA can be referred to as "uncapped RNA", for example. Uncapped RNA can better accumulate in the nucleus following transcription, since 5'-capped RNA is subject to nuclear export.

A ribozyme refers to one or more RNA sequences that form secondary, tertiary, and/or quaternary structure(s) that can cleave RNA at a specific site. A ribozyme includes a "self-cleaving ribozyme, or self-processing ribozyme" that is capable of cleaving RNA at a c/s-site relative to the ribozyme sequence (i.e., auto-catalytic, or self-cleaving). The general nature of ribozyme nucleolytic activity is known to the person skilled in the art. The of self-processing ribozymes in the production of guide-RNA's for RNA-guided nuclease systems such as CRISPR/Cas is *inter alia* described by Gao et al, 2014.

A nucleotide analogue or equivalent typically comprises a modified backbone. Examples of such backbones are provided by morpholino backbones, carbamate backbones, siloxane backbones, sulfide, sulfoxide and sulfone backbones, formacetyl and thioformacetyl backbones, methyleneformacetyl backbones, riboacetyl backbones, alkene containing backbones, sulfamate, sulfonate and sulfonamide backbones, methyleneimino and methylenehydrazino backbones, and amide backbones. It is further preferred that the linkage between a residue in a backbone does not include a phosphorus atom, such as a linkage that is formed by short chain alkyl or cycloalkyl internucleoside linkages, mixed heteroatom and alkyl or cycloalkyl internucleoside linkages, or one or more short chain heteroatomic or heterocyclic internucleoside linkages.

A preferred nucleotide analogue or equivalent comprises a Peptide Nucleic Acid (PNA), having a modified polyamide backbone (Nielsen, et al. (1991) Science 254, 1497-1500). PNA-based molecules are true mimics of DNA molecules in terms of base-pair recognition. The backbone of the PNA is composed of N-(2-aminoethyl)-glycine units linked by peptide bonds, wherein the nucleobases are linked to the backbone by methylene carbonyl bonds. An alternative backbone comprises a one-carbon extended pyrrolidine PNA monomer (Govindaraju and Kumar (2005) Chem. Commun, 495-497). Since the backbone of a PNA molecule contains no charged phosphate groups, PNA-RNA hybrids are usually more stable than RNA-RNA or RNA-DNA hybrids, respectively (Egholm et al (1993) Nature 365, 566-568).

A further preferred backbone comprises a morpholino nucleotide analog or equivalent, in which the ribose or deoxyribose sugar is replaced by a 6-membered morpholino ring. A most preferred nucleotide analog or equivalent comprises a phosphorodiamidate morpholino oligomer (PMO), in which the ribose or deoxyribose sugar is replaced by a 6-membered morpholino ring, and the anionic phosphodiester linkage between adjacent morpholino rings is replaced by a non-ionic phosphorodiamidate linkage.

A further preferred nucleotide analogue or equivalent comprises a substitution of at least one of the non-bridging oxygens in the phosphodiester linkage. This modification slightly destabilizes base-pairing but adds significant resistance to nuclease degradation. A preferred nucleotide analogue or equivalent comprises phosphorothioate, chiral phosphorothioate, phosphorodithioate, phosphotriester, aminoalkylphosphotriester, H-phosphonate, methyl and other alkyl phosphonate including 3'-alkylene phosphonate, 5'-alkylene phosphonate and chiral phosphonate, phosphinate, phosphoramidate including 3'-amino phosphoramidate and aminoalkylphosphoramidate, thionophosphoramidate, thionoalkylphosphonate, thionoalkylphosphotriester, selenophosphate or boranophosphate.

A further preferred nucleotide analogue or equivalent comprises one or more sugar moieties that are mono- or disubstituted at the 2', 3' and/or 5' position such as a -OH; -F; substituted or unsubstituted, linear or branched lower (C1-C10) alkyl, alkenyl, alkynyl, alkaryl, allyl, aryl, or aralkyl, that may be interrupted by one or more heteroatoms; O-, S-, or N-alkyl; O-, S-, or N-alkenyl; O-, S-or N-alkynyl; O-, S-, or N-allyl; O-alkyl-O-alkyl, -methoxy, -aminopropoxy; aminoxy, methoxyethoxy; - dimethylaminooxyethoxy; and -dimethylaminoethoxyethoxy. The sugar moiety can be a pyranose or derivative thereof, or a deoxypyranose or derivative thereof, preferably a ribose or a derivative thereof, or deoxyribose or derivative thereof. Such preferred derivatized sugar moieties comprise Locked Nucleic Acid (LNA), in which the 2'-carbon atom is linked to the 3' or 4' carbon atom of the sugar ring thereby forming a bicyclic sugar moiety. A preferred LNA comprises 2'-O,4'-C-ethylene-bridged nucleic acid (Morita et al. 2001. Nucleic Acid Res Supplement No. 1: 241-242). These substitutions render the nucleotide analogue or equivalent RNase H and nuclease resistant and increase the affinity for the target.

"Sequence identity" or "identity" in the context of the present invention of an amino acid- or nucleic acid-sequence is herein defined as a relationship between two or more amino acid (peptide, polypeptide, or protein) sequences or two or more nucleic acid (nucleotide, oligonucleotide, polynucleotide) sequences, as determined by comparing the sequences. In the art, "identity" also means the degree of sequence relatedness between amino acid or nucleotide sequences, as the case may be, as determined by the match between strings of such sequences. Within the present invention, sequence identity with a particular sequence preferably means sequence identity over the entire length of said particular polypeptide or polynucleotide sequence.

"Similarity" between two amino acid sequences is determined by comparing the amino acid sequence and its conserved amino acid substitutes of one peptide or polypeptide to the sequence of a second peptide or polypeptide. In a preferred embodiment, identity or similarity is calculated over the whole sequence (SEQ ID NO:) as identified herein. "Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988). Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, Wl. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

A polynucleotide according to the present invention is represented by a nucleotide sequence. A polypeptide according to the present invention is represented by an amino acid sequence. A nucleic acid construct according to the present invention is defined as a polynucleotide which is isolated from a naturally occurring gene or which has been modified to contain segments of polynucleotides which are combined or juxtaposed in a manner which would not otherwise exist in nature.

The sequence information as provided herein should not be so narrowly construed as to require inclusion of erroneously identified bases. The skilled person is capable of identifying such erroneously identified bases and knows how to correct for such errors.

All embodiments of the present invention, preferably refer to a cell, not to a cell-free *in vitro* system; in other words, the systems according to the invention are preferably cell systems, not cell-free *in vitro* systems.

In all embodiments of the present invention, e.g., the cell according to the present invention may be a haploid, diploid or polyploid cell.

A cell according to the invention is interchangeably herein referred as "a cell", "a cell according to the invention", "a host cell", and as "a host cell according to the invention"; said cell may be any cell, preferably a fungus, i.e. a yeast cell or a filamentous fungus cell. Preferably, the cell is deficient in an NHEJ (non-homologous end joining) component. Said component associated with NHEJ is preferably a homologue or orthologue of the yeast Ku70, Ku80, MRE11, RAD50, RAD51, RAD52, XRS2, SIR4, and/or LIG4. Alternatively, in the cell according to the invention NHEJ may be rendered deficient by use of a compound that inhibits RNA ligase IV, such as SCR7. Deficiency is defined elsewhere herein.

When the cell according to the invention is a yeast cell, a preferred yeast cell is from a genus selected from the group consisting of *Candida, Hansenula, Issatchenkia, Kluyveromyces, Pichia, Saccharomyces, Schizosaccharomyces, Yarrowia or Zygosaccharomyces;* more preferably a yeast host cell is selected from the group consisting of *Kluyveromyces lactis, Kluyveromyces lactis* NRRL Y-1140, *Kluyveromyces marxianus, Kluyveromyces. thermotolerans, Candida krusei, Candida sonorensis, Candida glabrata, Saccharomyces cerevisiae, Saccharomyces cerevisiae* CEN.PK113-7D, *Schizosaccharomyces pombe, Hansenula polymorpha, Issatchenkia orientalis, Yarrowia lipolytica, Yarrowia lipolytica* CLIB122, *Pichia stipidis* and *Pichia pastoris.*

The host cell according to the present invention is a filamentous fungal host cell. Filamentous fungi as defined herein include all filamentous forms of the subdivision *Eumycota* and *Oomycota* (as defined by Hawksworth et al., In, Ainsworth and Bisby's Dictionary of The Fungi, 8th edition, 1995, CAB International, University Press, Cambridge, UK).

The filamentous fungal host cell may be a cell of any filamentous form of the taxon *Trichocomaceae* (as defined by Houbraken and Samson in Studies in Mycology 70: 1-51.2011). In another preferred embodiment, the filamentous fungal host cell may be a cell of any filamentous form of any of the three families *Aspergillaceae, Thermoascaceae* and *Trichocomaceae,* which are accommodated in the taxon *Trichocomaceae.*

The filamentous fungi are characterized by a mycelial wall composed of chitin, cellulose, glucan, chitosan, mannan, and other complex polysaccharides. Vegetative growth is by hyphal elongation and carbon catabolism is obligatory aerobic. Filamentous fungal strains include, but are not limited to, strains of *Acremonium, Agaricus, Aspergillus, Aureobasidium, Chrysosporium, Coprinus, Cryptococcus, Filibasidium, Fusarium, Humicola, Magnaporthe, Mortierella, Mucor, Myceliophthora, Neocallimastix, Neurospora, Paecilomyces, Penicillium, Piromyces, Panerochaete, Pleurotus, Schizophyllum, Talaromyces, Rasamsonia, Thermoascus, Thielavia, Tolypocladium,* and *Trichoderma.* A preferred filamentous fungal host cell according to the present invention is from a genus selected from the group consisting of *Acremonium, Aspergillus, Chrysosporium, Myceliophthora, Penicillium, Talaromyces, Rasamsonia, Thielavia, Fusarium* and *Trichoderma;* more preferably from a species selected from the group consisting of *Aspergillus niger, Acremonium alabamense, Aspergillus awamori, Aspergillus foetidus, Aspergillus sojae, Aspergillus fumigatus, Talaromyces emersonii, Rasamsonia emersonii, Rasamsonia emersonii* CBS393.64, *Aspergillus oryzae, Chrysosporium lucknowense, Fusarium oxysporum, Mortierella alpina, Mortierella alpina* ATCC 32222, *Myceliophthora thermophila, Trichoderma reesei, Thielavia terrestris, Penicillium chrysogenum* and P. *chrysogenum* Wisconsin 54-1255(ATCC28089); even more preferably the filamentous fungal host cell according to the present invention is an *Aspergillus niger.* When the host cell according to the present invention is an *Aspergillus niger* host cell, the host cell preferably is CBS 513.88, CBS124.903 or a derivative thereof.

Several strains of filamentous fungi are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), Agricultural Research Service Patent Culture Collection, Northern Regional Research Center (NRRL), and All-Russian Collection of Microorganisms of Russian Academy of Sciences, (abbreviation in Russian - VKM, abbreviation in English - RCM), Moscow, Russia. Preferred strains as host cells according to the present invention are *Aspergillus niger* CBS 513.88, CBS124.903, *Aspergillus oryzae* ATCC 20423, IFO 4177, ATCC 1011, CBS205.89, ATCC 9576, ATCC14488-14491, ATCC 11601, ATCC12892, P. *chrysogenum* CBS 455.95, P. *chrysogenum* Wisconsin54-1255(ATCC28089), *Penicillium citrinum* ATCC 38065, *Penicillium chrysogenum* P2, *Thielavia terrestris* NRRL8126, *Rasamsonia emersonii* CBS393.64, *Talaromyces emersonii* CBS 124.902, *Acremonium chrysogenum ATCC* 36225 or ATCC 48272, *Trichoderma* reeseiATCC 26921 or ATCC 56765 or ATCC 26921, *Aspergillus sojae* ATCC1 1906, *Myceliophthora thermophila* C1, Garg 27K, VKM-F 3500 D, *Chrysosporium lucknowense* C1, Garg 27K, VKM-F 3500 D, ATCC44006 and derivatives thereof.

In the embodiments of the invention, the host cell may be an algae, a microalgae or a marine eukaryote. The host cell may be a *Labyrinthulomycetes* host cell, preferably of the order *Thraustochytriales,* more preferably of the family *Thraustochytriaceae,* more preferably a member of a genus selected from the group consisting of *Aurantiochytrium, Oblongichytrium, Schizochytrium, Thraustochytrium,* and *Ulkenia,* even more preferably Schizochytrium sp. ATCC# 20888.

Preferably, a host cell according to the invention has a modification, preferably in its genome which results in a reduced or no production of an undesired compound as defined herein if compared to the parent host cell that has not been modified, when analysed under the same conditions.

A modification can be introduced by any means known to the person skilled in the art, such as but not limited to classical strain improvement, random mutagenesis followed by selection. Modification can also be introduced by site-directed mutagenesis.

Modification may be accomplished by the introduction (insertion), substitution (replacement) or removal (deletion) of one or more nucleotides in a polynucleotide sequence. A full or partial deletion of a polynucleotide coding for an undesired compound such as a polypeptide may be achieved. An undesired compound may be any undesired compound listed elsewhere herein; it may also be a protein and/or enzyme in a biological pathway of the synthesis of an undesired compound such as a metabolite. Alternatively, a polynucleotide coding for said undesired compound may be partially or fully replaced with a polynucleotide sequence which does not code for said undesired compound or that codes for a partially or fully inactive form of said undesired compound. In another alternative, one or more nucleotides can be inserted into the polynucleotide encoding said undesired compound resulting in the disruption of said polynucleotide and consequent partial or full inactivation of said undesired compound encoded by the disrupted polynucleotide.

In an embodiment the host cell according to the invention comprises a modification in its genome selected from
a) a full or partial deletion of a polynucleotide encoding an undesired compound,
b) a full or partial replacement of a polynucleotide encoding an undesired compound with a polynucleotide sequence which does not code for said undesired compound or that codes for a partially or fully inactive form of said undesired compound.
c) a disruption of a polynucleotide encoding an undesired compound by the insertion of one or more nucleotides in the polynucleotide sequence and consequent partial or full inactivation
of said undesired compound by the disrupted polynucleotide.

This modification may for example be in a coding sequence or a regulatory element required for the transcription or translation of said undesired compound. For example, nucleotides may be inserted or removed so as to result in the introduction of a stop codon, the removal of a start codon or a change or a frame-shift of the open reading frame of a coding sequence. The modification of a coding sequence or a regulatory element thereof may be accomplished by site-directed or random mutagenesis, DNA shuffling methods, DNA reassembly methods, gene synthesis (see for example Young and Dong, (2004), Nucleic Acids Research 32(7*) or* Gupta et al. (1968), Proc. Natl. Acad. Sci USA, 60: 1338-1344; Scarpulla et al. (1982), Anal. Biochem. 121: 356-365*;* Stemmeret al. (1995), Gene 164: 49-53), or PCR generated mutagenesis in accordance with methods known in the art. Examples of random mutagenesis procedures are well known in the art, such as for example chemical (NTG for example) mutagenesis or physical (UV for example) mutagenesis. Examples of site-directed mutagenesis procedures are the QuickChange^{™} site-directed mutagenesis kit (Stratagene Cloning Systems, La Jolla, CA), the 'The Altered Sites^{®} II in vitro Mutagenesis Systems' (Promega Corporation) or by overlap extension using PCR as described in Gene. 1989 Apr 15; 77(1):51-9. (Ho SN, Hunt HD, Horton RM, Pullen JK, Pease LR "Site-directed mutagenesis by overlap extension using the polymerase chain reaction") or using PCR as described in Molecular Biology: Current Innovations and Future Trends. (Eds. A.M. Griffin and H.G.Griffin. ISBN 1-898486-01-8; 1995 Horizon Scientific Press, PO Box 1, Wymondham, Norfolk, U.K.).

Preferred methods of modification are based on recombinant genetic manipulation techniques such as partial or complete gene replacement or partial or complete gene deletion.

For example, in case of replacement of a polynucleotide, nucleic acid construct or expression cassette, an appropriate DNA sequence may be introduced at the target locus to be replaced. The appropriate DNA sequence is preferably present on a cloning vector. Preferred integrative cloning vectors comprise a DNA fragment, which is homologous to the polynucleotide and / or has homology to the polynucleotides flanking the locus to be replaced for targeting the integration of the cloning vector to this pre-determined locus. In order to promote targeted integration, the cloning vector is preferably linearized prior to transformation of the cell. Preferably, linearization is performed such that at least one but preferably either end of the cloning vector is flanked by sequences homologous to the DNA sequence (or flanking sequences) to be replaced. This process is called homologous recombination and this technique may also be used in order to achieve (partial) gene deletion.

For example a polynucleotide corresponding to the endogenous polynucleotide may be replaced by a defective polynucleotide; that is a polynucleotide that fails to produce a (fully functional) polypeptide. By homologous recombination, the defective polynucleotide replaces the endogenous polynucleotide. It may be desirable that the defective polynucleotide also encodes a marker, which may be used for selection of transformants in which the nucleic acid sequence has been modified.

Alternatively or in combination with other mentioned techniques, a technique based on recombination of cosmids in an E. *coli* cell can be used, as described in: A rapid method for efficient gene replacement in the filamentous fungus Aspergillus nidulans (2000) Chaveroche, M-K., Ghico, J-M. and d'Enfert C; Nucleic acids Research, vol 28, no 22*.*

Alternatively, modification, wherein said host cell produces less of or no protein such as the polypeptide having amylase activity, preferably α-amylase activity as described herein and encoded by a polynucleotide as described herein, may be performed by established anti-sense techniques using a nucleotide sequence complementary to the nucleic acid sequence of the polynucleotide. More specifically, expression of the polynucleotide by a host cell may be reduced or eliminated by introducing a nucleotide sequence complementary to the nucleic acid sequence of the polynucleotide, which may be transcribed in the cell and is capable of hybridizing to the mRNA produced in the cell. Under conditions allowing the complementary anti-sense nucleotide sequence to hybridize to the mRNA, the amount of protein translated is thus reduced or eliminated. An example of expressing an antisense-RNA is shown in Appl. Environ. Microbiol. 2000 Feb; 66(2):775-82. (Characterization of a foldase, protein disulfide isomerase A, in the protein secretory pathway of Aspergillus niger. Ngiam C, Jeenes DJ, Punt PJ, Van Den Hondel CA, Archer DB*)* or *(*Zrenner R, Willmitzer L, Sonnewald U. Analysis of the expression of potato uridinediphosphate-glucose pyrophosphorylase and its inhibition by antisense RNA. Planta. (1993); 190(2):247-52*.).*

A modification resulting in reduced or no production of undesired compound is preferably due to a reduced production of the mRNA encoding said undesired compound if compared with a parent microbial host cell which has not been modified and when measured under the same conditions. A modification which results in a reduced amount of the mRNA transcribed from the polynucleotide encoding the undesired compound may be obtained via the RNA interference (RNAi) technique (Mouyna et al., 2004). In this method identical sense and antisense parts of the nucleotide sequence, which expression is to be affected, are cloned behind each other with a nucleotide spacer in between, and inserted into an expression vector. After such a molecule is transcribed, formation of small nucleotide fragments will lead to a targeted degradation of the mRNA, which is to be affected. The elimination of the specific mRNA can be to various extents. The RNA interference techniques described in e.g. WO2008/053019, WO2005/05672A1 and WO2005/026356A1,.

A modification which results in decreased or no production of an undesired compound can be obtained by different methods, for example by an antibody directed against such undesired compound or a chemical inhibitor or a protein inhibitor or a physical inhibitor (Tour O. et al, (2003) Nat. Biotech: Genetically targeted chromophore-assisted light inactivation. Vol.21. no. 12:1505-1508) or peptide inhibitor or an anti-sense molecule or RNAi molecule (R.S. Kamath_et al, (2003) Nature: Systematic functional analysis of the Caenorhabditis elegans genome using RNAi. Vol. 421, 231-237).

In addition of the above-mentioned techniques or as an alternative, it is also possible to inhibiting the activity of an undesired compound, or to re-localize the undesired compound such as a protein by means of alternative signal sequences (Ramon de Lucas, J., Martinez O, Perez P., Isabel Lopez, M., Valenciano, S. and Laborda, F. The Aspergillus nidulans carnitine carrier encoded by the acuH gene is exclusively located in the mitochondria. FEMS Microbiol Lett. 2001 Jul 24; 201(2):193-8.) or retention signals (Derkx, P. M. and Madrid, S. M. The foldase CYPB is a component of the secretory pathway of Aspergillus niger and contains the endoplasmic reticulum retention signal HEEL. Mol. Genet. Genomics. 2001 Dec;266(4):537-545), or by targeting an undesired compound such as a polypeptide to a peroxisome which is capable of fusing with a membrane-structure of the cell involved in the secretory pathway of the cell, leading to secretion outside the cell of the polypeptide (e.g. as described in WO2006/040340).

Alternatively or in combination with above-mentioned techniques, decreased or no production of an undesired compound can also be obtained, e.g. by UV or chemical mutagenesis (Mattern, I.E., van Noort J.M., van den Berg, P., Archer, D. B., Roberts, I.N. and van den Hondel, C. A., Isolation and characterization of mutants of Aspergillus niger deficient in extracellular proteases. Mol Gen Genet. 1992 Aug; 234(2):332-6.) or by the use of inhibitors inhibiting enzymatic activity of an undesired polypeptide as described herein (e.g. nojirimycin, which function as inhibitor for β-glucosidases (Carrel F.L.Y. and Canevascini G. Canadian Journal of Microbiology (1991) 37(6): 459-464; Reese E.T., Parrish F.W. and Ettlinger M. Carbohydrate Research (1971) 381-388)). In an embodiment of the invention, the modification in the genome of the host cell according to the invention is a modification in at least one position of a polynucleotide encoding an undesired compound.

A deficiency of a cell in the production of a compound, for example of an undesired compound such as an undesired polypeptide and/or enzyme is herein defined as a mutant microbial host cell which has been modified, preferably in its genome, to result in a phenotypic feature wherein the cell: a) produces less of the undesired compound or produces substantially none of the undesired compound and/or b) produces the undesired compound having a decreased activity or decreased specific activity or the undesired compound having no activity or no specific activity and combinations of one or more of these possibilities as compared to the parent host cell that has not been modified, when analysed under the same conditions.

Preferably, a modified host cell according to the invention produces 1% less of the un-desired compound if compared with the parent host cell which has not been modified and measured under the same conditions, at least 5% less of the un-desired compound, at least 10% less of the un-desired compound, at least 20% less of the un-desired compound, at least 30% less of the un-desired compound, at least 40% less of the un-desired compound, at least 50% less of the un-desired compound, at least 60% less of the un-desired compound, at least 70% less of the un-desired compound, at least 80% less of the un-desired compound, at least 90% less of the un-desired compound, at least 91% less of the un-desired compound, at least 92% less of the un-desired compound, at least 93% less of the un-desired compound, at least 94% less of the un-desired compound, at least 95% less of the un-desired compound, at least 96% less of the un-desired compound, at least 97% less of the un-desired compound, at least 98% less of the un-desired compound, at least 99% less of the un-desired compound, at least 99.9% less of the un-desired compound, or most preferably100% less of the un-desired compound.

A reference herein to a patent document or other matter which is given as prior art is not to be taken as an admission that that document or matter was known or that the information it contains was part of the common general knowledge as at the priority date of any of the claims.

The sequence information as provided herein should not be so narrowly construed as to require inclusion of erroneously identified bases. The skilled person is capable of identifying such erroneously identified bases and knows how to correct for such errors.

The present invention is further illustrated by the following examples:

### EXAMPLES

In the following Examples, various embodiments of the invention are illustrated. From the above description and these Examples, one skilled in the art can make various changes and modifications of the disclosure to adapt it to various usages and conditions.

To enable genome precision engineering in a cell using the RNA-guided CRISPR/Cas9 system, the essential components being the Cas9 protein and the crRNA-tracrRNA fusion transcript (referred as guide-RNA or gRNA), should be expressed at the same time within the cell. Similarly, to enable expression regulation such as by CRISPR interference (CRISPRi), the dCas9 protein must be expressed concurrently with a gRNA (Qi et al. 2013). Other uses of the CRISPR/Cas system would also necessitate the concurrent expression of the Cas9 (or variant of Cas9) and gRNA. In a similar way, for other RNA-guided endonucleases, the guide Cpf1 and de corresponding crRNA or crRNA array (Zetsche et al, 2015).

### Example 1: Construction of dCas9 / SNR52 promoter / T7 promoter - T7 RNA polymerase test strains and gRNA expression vectors

The parent test strain used for all experiments was CEN.PK113-7D (MATa *URA3 HIS3 LEU2 TRP1 MAL2-8 SUC2).* Strain CEN.PK113-7D is available from the EUROSCARF collection (www.euroscarf.de, Frankfurt, Germany) or from the Centraal Bureau voor Schimmelcultures (Utrecht, the Netherlands, deposit number CBS 8340). The origin of the CEN.PK family of strains is described by van Dijken *et al., 2000.* Test strains AG1 and AG2 were constructed by transformation of expression constructs that were amplified by PCR and *in vivo* assembly in *Saccharomyces cerevisiae* (described in detail below). Integration of DNA into the genome was performed at the INT1 locus (located at the non-coding region between NTR1 (YOR071c) and GYP1 (YOR070c) located on chromosome XV). All gRNA expression vectors were assembled *in vivo* by transformation of PCR fragments in the AG1 and AF2 test strains, resulting in guide RNA expression vectors pRN1120-AG1 and pAG701.

### integration of expression elements by in vivo assembly into genomic DNA

PCR was used to produce three linear fragments that were assembled *in vivo* and integrated into genomic DNA of strain CEN.PK113-7D, resulting in test strains AG1 and AG2. Primers were designed to produce about 50-bp overlaps with adjacent pieces and 5' and 3' connector sequences, which had homology to the INT1 locus. Forward and reverse PCR primers for the 5' fragment are show in in SEQ ID NO: 1 and 2 while those for the 3' fragment are shown in SEQ ID NO: 3 and 4. The presence of highly homologous (about 50-bp overlaps) pieces and DNA flank sequences allow assembly to one stretch of DNA by *in vivo* homologous recombination (gap repair, Orr-Weaver *et al.,* 1983) at the desired location and in the desired order into the genomic DNA.

PCR fragments for the donor DNA expression cassette sequences were generated using Phusion DNA polymerase (New England Biolabs, USA) according to manufacturer's instructions. All PCR fragments were purified using DNA Clean & Concentrator^{™}-5 kit (distributed by Baseclear Lab Products, Leiden, the Netherlands), according to manufacturer's instructions. The 5' and 3' fragments were generated by PCR using genomic DNA (isolated from the yeast strain CEN.PK113-7D using the lithium acetate SDS method (Lõoke et al., 2011)) as a template.

### Strain descriptions

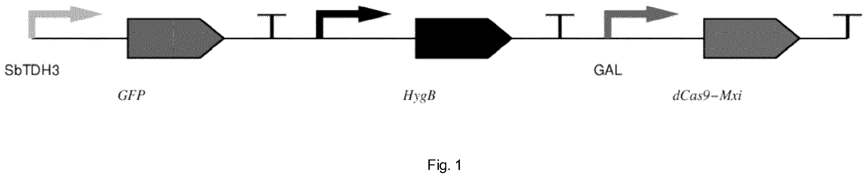

The DNA sequences present in strain AG1 are graphically depicted in Figure 1 and the final DNA sequence, as obtained after *in vivo* assembly into genomic DNA of strain CEN.PK113-7D, is provided in SEQ ID NO: 5. Strain AG1 constitutively expresses GFP. The promoter driving GFP expression is the TDH3 promoter from *Saccharomyces bayanus* (SbTDH3). Strain AG1 expresses dCas9-Mxi1 from a galactose-inducible promoter (GAL1p). The dCas9-Mxi1 nucleotide sequence was taken from Gilbert *et al.,* 2013. Stain AG1 contains a Hygromycin selection marker that was initially used to select for the properly assembled strain; however, hygromycin was not used for selection in the repression assay, described in Example 2.

The DNA sequences present in strain AG2 are graphically depicted in Figure 2 and the final DNA sequence, as obtained after *in vivo* assembly into genomic DNA of strain CEN.PK113-7D, is provided in SEQ ID NO: 6. Strain AG2 constitutively expresses GFP. The promoter driving GFP expression is the TDH3 promoter from *Saccharomyces bayanus* (SbTDH3). Strain AG2 expresses dCas9-Mxi1 and T7-RNAp from a galactose-inducible promoter (GAL1p). The two coding sequences dCas9-Mxi1 and T7 RNA polymerase (T7-RNAp) are separated by a viral T2A sequence that enables co-expression of multiple proteins from a single promoter. The T2A sequence originates from *Thosea asigna* virus. The amino acids SGS, which are said to improve cleavage efficiency (Kim *et al.,* 2011), were not encoded by the T2A nucleotide sequence used in this Example. The dCas9-Mxi1 nucleotide sequence was taken from Gilbert *et al.,* 2013. The T7-RNAp nucleotide sequence was directly PCR amplified from the *Escherichia coli* BL21-DE3 strain from New England Biolabs (product ID: C2527I). Strain AG2 contains a Hygromycin selection marker that was initially used to select for the properly assembled strain; however, hygromycin was not used for selection in the repression assays, described in Example 2 and Example 3.

### Vector descriptions

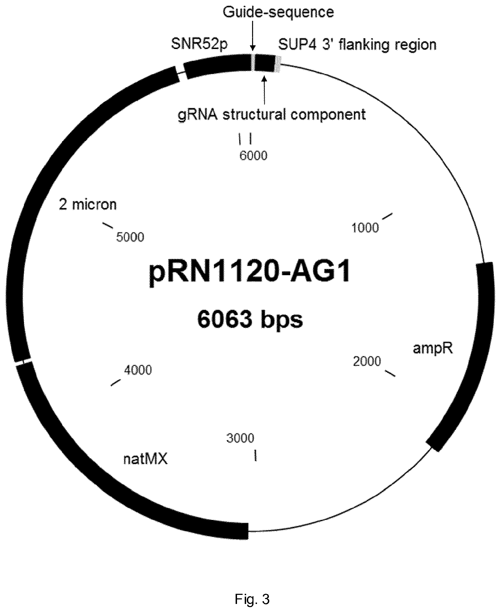

The parent vector for the experiments in the AG1 strain was pRN1120-AG1 (SEQ ID NO: 7, Figure 3), which contains a 2-micron yeast origin of replication and a NatMX antibiotic selection marker. The vector also contains a guide RNA expression cassette consisting of a SNR52 promoter, a guide-sequence targeting the SbTDH3 promoter that was used for constitutive GFP expression in strain AG1, a guide RNA structural component, and a SUP4 terminator as described in DiCarlo *et al.,* 2013.

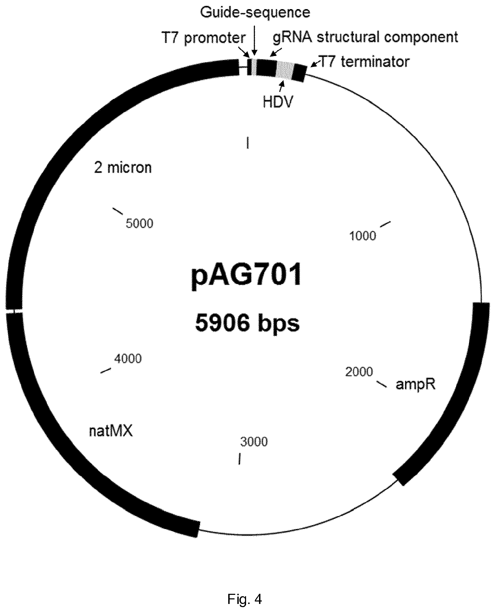

The parent vector for the experiments in the AG2 strain was pAG701 (SEQ ID NO: 8, Figure 4) which contains a 2-micron yeast origin of replication and a NatMX antibiotic selection marker. The T7 promoter gRNA expression vector was constructed by replacing the SNR52 promoter from the pRN1120-AG1 with a T7 promoter. The SUP4 terminator was replaced by a Hepatitis Delta Virus ribozyme (HDVr) and a T7 terminator, resulting in vector pAG701. The HDVr is a self-cleaving RNA element that corrects for run-on transcription form T7 caused by weak termination (Szafraniec *et al.* 2012). The guide-sequence and guide RNA structural component remained intact. The gRNA targets the SbTDH3 promoter that was used for constitutive GFP expression in strain AG2.

All gRNA expression vectors were constructed using PCR to generate linear DNA fragments and *in vivo* assembly in *Saccharomyces cerevisiae* (described below).

To test repression efficiency, a gRNA targeting the (SbTDH3) promoter was designed using the gRNA design tool on the internet (benchling.com) (SEQ ID NO: 9). All repression results were normalized to a scrambled gRNA that targets a random 20-nt DNA sequence that is not found in the yeast genome (SEQ ID NO: 10).

### In vivo vector assembly

PCR was used to generate linear fragments of the pieces of the vectors. PCR was performed using primers that confer a 50-bp overlap with adjacent pieces. When transformed the pieces assemble into a circular vector by *in vivo* homologous recombination (gap repair, Orr-Weaver et al., 1983), which allows selection of transformants on nourseothricin.

The T7 gRNA transcription unit (part of SEQ ID NO: 8) was ordered as a synthetic DNA cassette (gBlock) at Integrated DNA Technologies, Leuven, Belgium. The cassette contained 50-bp homology with the parent vector, which was PCR amplified without the SNR52 promoter, and was assembled by *in vivo* recombination in yeast. Linear DNA fragments required for assembly of vector pRN1120-AG1 were transformed to test strain AG1. Linear DNA fragments required for assembly of vector pAG701 were transformed to test strain AG2.

### Transformation conditions

Vector backbones and linear DNA pieces (for integration into genomic DNA or assembly into vectors) were transformed into S. *cerevisiae* strain CEN-PK1137D using the LiAc/salmon sperm (SS) carrier DNA/PEG method (Gietz and Woods, 2002). In the transformation mixture equimolar concentrations of each DNA piece were used (normalized to 1 microgram of the shortest DNA piece used in the reaction). The transformation mixture was plated on YPD-agar (10 grams per litre of yeast extract, 20 grams per litre of peptone, 20 grams per litre of dextrose, 20 grams per litre of agar). For construction of strains AG1 and AG2 200 micrograms per ml Hygromycin (Sigma Aldrich, Zwijndrecht, the Netherlands) was added to the YPD-agar. For yeast *in vivo* vector assembly 100 micrograms per ml nourseothricin (Sigma Aldrich, Zwijndrecht, the Netherlands) was added to the agar. After three to four days of growth at 30°C, colonies appeared on the transformation plate.

### Example 2: Efficiency of T7-produced gRNAs

### Repression Assay

Strains AG1 and AG2 were transformed with linear DNA fragments to allow *in vivo* assembly to generate vectors pRN1120-AG1 (Figure 3) and pAG701 (Figure 4). Three colonies from the transformations plates were picked and cultured for 16 hours in 5 ml of YPD medium (10 grams per litre of yeast extract, 20 grams per litre of peptone, 20 grams per litre of dextrose) supplemented with nourseothricin selection (100 micrograms per ml). The cultures were subsequently diluted 1/200 into in YEP medium (10 grams per litre of yeast extract, 20 grams per litre of peptone) containing 2% galactose (Sigma Aldrich, Zwijndrecht, the Netherlands) supplemented with nourseothricin selection (100 micrograms per ml) to induce expression of dCas9-Mxi1 in the AG1 transformants and dCas9-Mxi1 and T7-RNA polymerase (T7-RNAp) expression in the AG2 transformants.

After 20 hours of growth on galactose medium, cells were diluted 1/20 into sterile water. The diluted cultures were run on a Tecan F200 plate reader (Tecan Trading AG, Switzerland) to quantify GFP fluorescence (excitation 480nm, emission 515nm) and OD600. The diluted cultures were also run on a MACSQuant VYB flow cytometer (Miltenyi Biotec, Germany) to quantify GFP fluorescence on a per cell basis and to observe cell population distributions.

To show functionality of T7-produced gRNAs compared to SNR52-produced gRNAs we directly compared GFP fluorescence, as a measure of dCas9-Mxi1 activity. Vector pRN1120-AG1, in which a gRNA targeting the SbTDH3 promoter was expressed from the SNR52 promoter, was *in vivo* assembled in strain AG1 as described in Example 1. Vector pAG701, in which a gRNA targeting the same site (guide-sequence indicated in SEQ ID NO: 9) on the SbTDH3 promoter was expressed from a T7 promoter, was *in vivo* assembled in strain AG2 as described in Example 1. Scrambled gRNA vectors for both the SNR52 and T7 systems were *in vivo* assembled in the appropriate strain for repression normalization. The scrambled gRNA vector is a control for vector maintenance load and promoter load on the strain, as the scrambled gRNA (guide-sequence indicated in SEQ ID NO: 10) will be produced in the cell, but will not target the SbTDH3 promoter driving GFP expression.

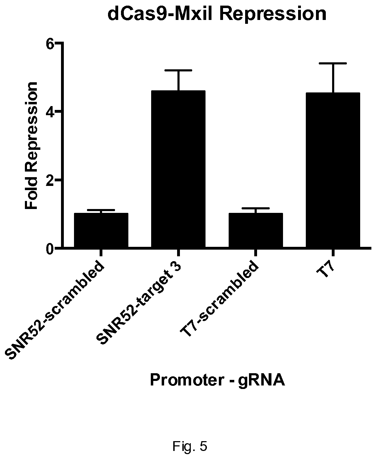

Fold repression (calculated using flow cytometry data) for each vector tested is depicted in Figure 5. Scrambled controls for both test strains AG1 and AG2 expressed GFP at the same level. Fold repression achieved by targeting the same position on the promoter driving GFP expression using different gRNA production methods is nearly identical. Fold repression was calculated by dividing the geometric mean of the targeting strain by that of the corresponding scrambled strain.

The results indicated that GFP expression can be repressed in this system using the SNR52 promoter for the expression of guide RNA. In addition, the results indicate that using the T7 promoter to induce expression of guide RNA, also resulted in repression of the GFP signal using the system described above, indicating the T7 RNA polymerase and the T7 promoter used to express guide RNA are functional in S. *cerevisiae.*

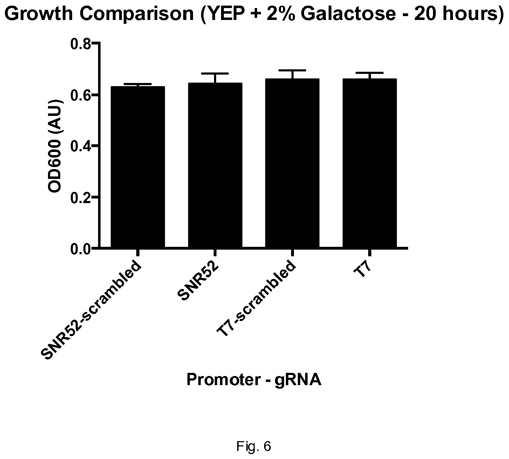

Figure 6 depicts the difference in growth between all strains. The expression of T7-RNAp has no effect on cell growth. The expression of a targeting gRNA compared to a scrambled gRNA (that targets a random sequence) also has no effect on growth.

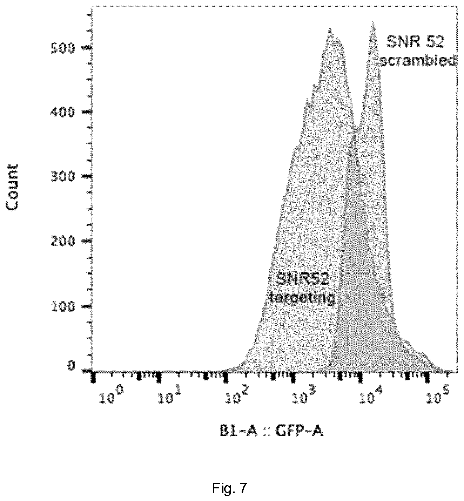

Figure 7 depicts the cell population as determined by flow cytometry of the SNR52-produced gRNA targeting the SbTDH3 promoter strain compared to the SNR52 scrambled strain. Both cell populations are mono-modal (a single peak for each cell population is observed), indicating strain and vector stability.

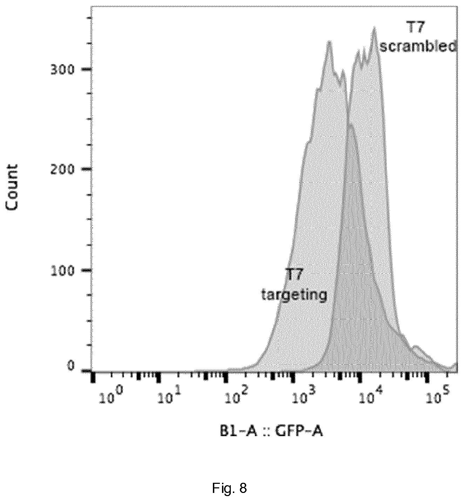

Figure 8 depicts the cell population (flow cytometry) of the T7-produced gRNA targeting the SbTDH3 promoter strain compared to the T7-produced scrambled strain. Both cell populations are mono-modal indicating strain and vector stability.

### Example 3: Modulating T7-produced gRNA expression

T7 promoters of different strengths, as characterized in E. *coli* (Jones *et al.,* 2015), were used to express gRNAs and the resulting GFP gene knockdown levels were quantified. Three different T7 promoters with high, medium, and low transcription rates were used (SEQ ID NO: 11, 12, 13), which replaced the T7 promoter sequence that are indicated in bold in SEQ ID NO: 8. The T7 promoters differ by 3-5 bp.

Different PCR fragments that allow *in vivo* assembly into a functional vector were transformed into strain AG2 as described in Example 1, resulting in variants of vector pAG701 (Figure 4, SEQ ID NO: 8) in which the T7 promoter was replaced by the sequences indicated in SEQ ID NO: 11, 12 and 13. Three individual transformants were grown and analyzed for GFP expression as described under repression assay in Example 2.

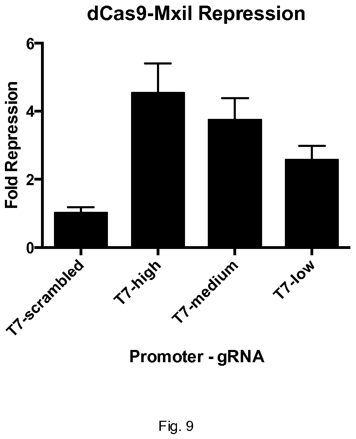

The results, depicted in Figure 9, indicate that the T7 promoter strength as characterized in E. *coli* (Jones *et al.,* 2015) correlated with target gene repression when used to express gRNAs in S. *cerevisiae.* All repression results were normalized to a scrambled gRNA expressed from the strong T7 promoter that targets a random 20-nt DNA sequence (guide sequence indicated in SEQ ID NO: 10). The results demonstrate that T7 promoters of varying strength can be used for functional expression of guide RNA in S. *cerevisiae* in combination with expression of T7 RNA polymerase.

The ease and versatility of tuning T7 promoter strengths by building degenerate base libraries (Temme et al. 2012) or selecting pre-characterized T7 promoters allows for precise gene expression tuning/regulation via modulation of gRNA expression levels. This invention may have applications in metabolic pathway optimization and genome editing if used with the Cas9 nuclease.

### Example 4: Functionality guide RNA under control of T7 promoter in S. cerevisiae

This example describes the functionality of the CRISPR Cas system in S. *cerevisiae* when making use of the T7 expression system for guide RNA expression. All gRNA expression vectors were assembled *in vivo* by transformation of PCR fragments comprising the guide RNA cassette under control of T7 promoter. S. *cerevisiae* strains CSN007 and CSN008 in which Cas9 as well as T7 RNAP is pre-expressed were used in transformation and thereby used for evaluation of the functionality of the T7 expression system for guide RNA expression.

### Construction of Cas9 and T7 RNAP expressing Saccharomyces cerevisiae strains CSN007 and CSN008

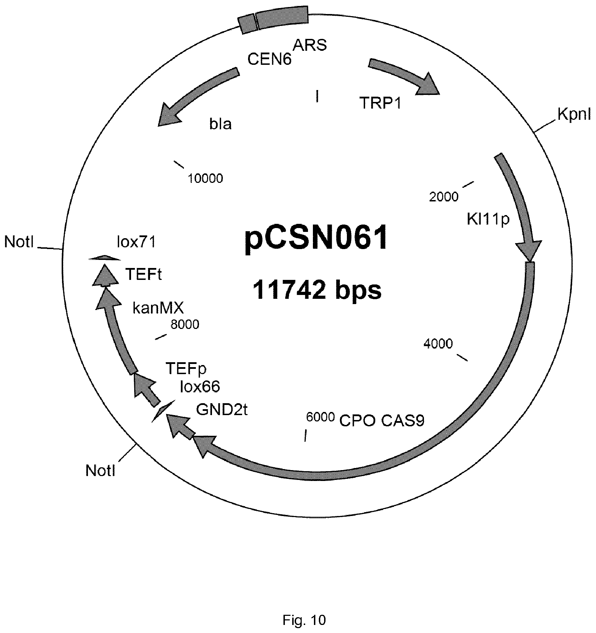

Yeast vector pCSN061 is a single copy vector (CEN/ARS) that contains a CAS9 expression cassette consisting of a CAS9 codon optimized variant expressed from the KI11 promoter (*Kluyveromyces lactis* promoter of KLLA0F20031g) and the S. *cerevisiae* GND2 terminator, and a functional KanMX marker cassette conferring resistance against G418. The CAS9 expression cassette was *Kpn*I/*Not*I ligated into pRS414 (Sikorski and Hieter, 1989), resulting in intermediate vector pCSN004. Subsequently, a functional expression cassette conferring G418 resistance (see http://www.euroscarf.de) was *Not*I restricted from vector pUG7-KanMX and Notl ligated into pCSN004, resulting in vector pCSN061 that is depicted in Figure 10 and the sequence is set out in SEQ ID NO: 15.

The vector pCSN061 containing the Cas9 expression cassette was Spel/Sacll restricted and 100 ng of the linearized pCSN061 vector was transformed to S. *cerevisiae* strain CEN.PK113-7D (MATa *URA3 HIS3 LEU2 TRP1 MAL2-8 SUC2*) with 105 ng of the T7 RNAP expression cassette (SEQ ID NO: 17 or SEQ ID NO: 18). The T7 RNAP expression cassette was ordered as synthetic DNA (ordered at DNA2.0, Menlo Park, CA, USA) and comprises the codon pair optimized T7 RNAP ORF (SEQ ID NO: 19) for expression in S. *cerevisiae* under control of Ptdh3 (SEQ ID NO: 20) or Penol (SEQ ID NO: 21) for a high or medium expression level of the T7 RNAP gene. To end the transcription of the gene the Tenol terminator (SEQ ID NO: 22) is placed after the stop codon. The T7 RNAP expression cassette is flanked on the 5' side with 64 bp homology on the 5' side and on the 3' side with 59 bp homology with the linear pCSN061 to assemble into a circular vector, pCSN070 or pCSN071 by *in vivo* homologous recombination (gap repair, Orr-Weaver et al., 1983). Transformation method used is the LiAc/salmon sperm (SS) carrier DNA/PEG method (Gietz and Woods, 2002) and transformants were selected on YPD-agar (10 grams per liter of yeast extract, 20 grams per liter of peptone, 20 grams per liter of dextrose, 20 grams per liter of agar) containing 200 microgram (µg) G418 (Sigma Aldrich, Zwijndrecht, the Netherlands) per ml. After two to four days of growth at 30°C transformants appeared on the transformation plate. Out of the resulting transformants 8 colonies were tested by PCR for correct assembly of the T7 RNAP expression cassette in pCSN061 plasmid backbone. Total DNA of the transformants was isolated as described by Lõoke et al., 2011 and was used as template in the PCR reaction. The primers used to confirm the transition of the pCSN061 plasmid backbone to the T7 RNAP are SEQ ID NO: 23 and SEQ ID NO: 24 for the 5' transition and SEQ ID NO: 25 and SEQ ID NO: 26 for the 3' transition of the T7 RNAP expression cassette to the pCSN061 backbone. The PCR reaction was performed using MyTaqTM Red Mix (Catno BIO-25044, Bioline - Germany) according to manufacturer's instructions and a PCR program known to a person skilled in the art. Correct asssembly was demonstrated by a fragment of 934 bp for 5' transition and 467 bp for 3' transition. Resulting PCR fragments were analyzed on a 0.8% agarose gel using 1x TAE buffer (50x TAE (Tris/ Acetic Acid/ EDTA), 1 liter, Cat no. 1610743, BioRad, The Netherlands) and 520-Nancy (Cat no. 01494, Sigma Aldrich, Germany) to stain the dsDNA.

Out of the 8 transformants tested for correct assembly of the T7 RNAP expression cassette in pCSN061 backbone, 100 % were correct in case of Penol-T7RNAP-Tenol and 87.5% was correct in case of Ptdh3-T7RNAP-Tenol assembly.

Transformants for which the presence of T7 RNAP was confirmed were designated CSN007 in case of Ptdh3 controlled T7 RNAP expression (plasmid pCSN070, SEQ ID NO: 27) and CSN008 in case of Penol controlled T7 RNAP expression (plasmid pCSN071 , SEQ ID NO: 28).

Strains CSN007 and CSN008 were used in transformation for evaluation of the functionality of T7 system for guide RNA expression in CRISPR Cas system.

### T7 controlled guide RNA expression cassettes

T7 promoter guide RNA expression cassettes were ordered as synthetic DNA (gBlocks) at Integrated DNA Technologies (IDT, Leuven, Belgium) homology to the pRN1120 on the 5' and 3' side for in vivo assembly were included. An overview of the sequences is provided in Table 1. The T7 promoter guide RNA consists a T7 promoter, a guide-sequence (also referred to as genomic target sequence; SEQ ID NO: 29), the gRNA structural component DiCarlo *et al.,* 2013 (SEQ ID NO: 61) and the Hepatitis Delta Virus ribozyme (HDVr) (SEQ ID NO: 30) and a T7 terminator (SEQ ID NO: 31). The HDVr is a self-cleaving RNA element that corrects for run-on transcription form T7 caused by weak termination (Szafraniec *et al.* 2012). For guide RNA expression a set of T7 promoters was tested with variable strength, the weak T7 promoter has SEQ ID NO: 32, the medium and strong T7 promoter are presented in SEQ ID NO: 33 and SEQ ID NO: 34 respectively. Also the wildtype T7 promoter was included in the set of tested T7 promoters and has SEQ ID NO: 35.

The INT1 locus is targeted by all 4 guide RNA cassettes for integration of the donor YFP expression cassette / ds break. The INT1 integration site is located in the non-coding region between NTR1 (YOR071c) and GYP1 (YOR070c), located on chromosome XV.

An overview of the PCR reactions performed to obtain the T7 controlled guide RNA expression cassettes that are to be used in transformation is presented in Table 1. PCR reactions were performed using PrimeStar GXL DNA polymerase (Takara / Catno. R050A) according to supplier's instructions and a PCR program known to a person skilled in the art. The generated T7 controlled guide RNA expression cassette PCR fragments were purified using the NucleoSpin Gel and PCR Clean-up kit (Machery-Nagel, distributed by Bioké, Leiden, the Netherlands) according to manufacturer's instructions. Subsequently, DNA concentrations of purified T7 controlled guide RNA fragments were measured using a NanoDrop (ND-1000 Spectrophotometer, Thermo Scientific, Bleiswijk, the Netherlands).

**Table 1. Overview of the sequences of the T7 controlled guide RNA sequences used in transformation. The gBlocks that were ordered at IDT (Leuven, Belgium) were used as a template for PCR using the primers indicated in this Table 1 in order to obtain T7 controlled guide RNA fragments used in the transformation experiments.**

| **Target** | **T7 promoter strength** | **guide RNA expression cassette** | **Guide sequence (genomic target sequence)** | **Primers used to obtain T7 controlled guide RNA fragment** | **Sequence T7 controlled guide RNA fragment** |
|---|---|---|---|---|---|
| INT1 site | Strong | SEQ ID NO: 36 | SEQ ID NO: 29 | SEQ ID NO: 40 | SEQ ID NO: 42 |
| | | | | SEQ ID NO: 41 | |
| INT1 site | Medium | SEQ ID NO: 37 | SEQ ID NO: 29 | SEQ ID NO: 40 | SEQ ID NO: 43 |
| | | | | SEQ ID NO: 41 | |
| INT1 site | Weak | SEQ ID NO: 38 | SEQ ID NO: 29 | SEQ ID NO: 40 | SEQ ID NO: 44 |
| | | | | SEQ ID NO: 41 | |
| INT1 site | Wild Type | SEQ ID NO: 39 | SEQ ID NO: 29 | SEQ ID NO: 40 | SEQ ID NO: 45 |
| | | | | SEQ ID NO: 41 | |

### pRN1120 vector construction (multi-copy expression vector, NatMX marker)

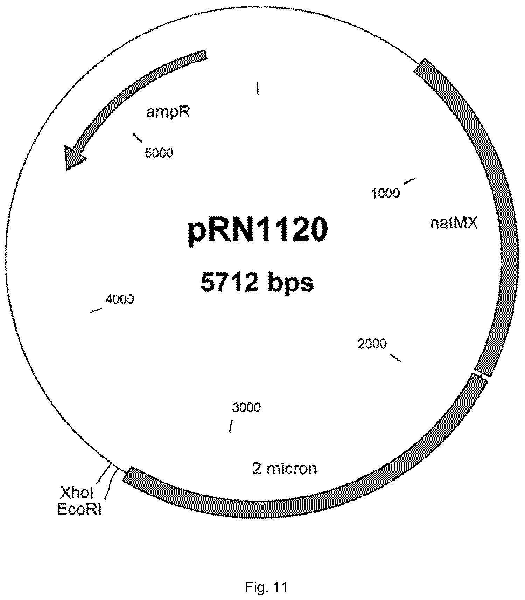

Yeast vector pRN1120 is a multi-copy vector (2 micron) that contains a functional NatMX marker cassette conferring resistance against nourseothricin. The backbone of this vector is based on pRS305 (Sikorski and Hieter, 1989), and includes a functional 2 micron ORI sequence and a functional NatMX marker cassette (see http://www.euroscarf.de). Vector pRN1120 is depicted in Figure 11 and the sequence is set out in SEQ ID NO: 16.

### integration sites

The INT1 integration site is located in the non-coding region between NTR1 (YOR071c) and GYP1 (YOR070c), located on chromosome XV.

### 100 bp ssODN flank sequences

To target the integration of the donor YFP expression cassette (SEQ ID NO: 46), so called flanks of 100 bp were supplied in the transformation. These left flank (LF) and right flank (RF) sequences have 50 bp homology with the 5'-terminus and 3'-terminus of the YFP donor cassette, and 50 bp homology with the genome. Each of the 100 bp flanks is comprised of 2 complementary single stranded DNA (ssODN) fragments. The LF consists of SEQ ID NO: 46 and SEQ ID NO: 47 and the RF consists of SEQ ID NO: 48 and SEQ ID NO: 49. By integration of integration of the YFP cassette a stretch of 1 kbp genomic DNA is knocked out at the INT1 locus.

### 500 bp double-stranded flank sequences

To target the integration of the donor YFP expression cassette (SEQ ID NO: 50), so called flanks of 577 or 581 bp were supplied in the transformation. These left flank (LF) and right flank (RF) sequences have 50 bp homology with the 5'-terminus and 3'-terminus of the YFP donor cassette, and 527 bp (LF) and 531 bp (RF) homology with the genome. These flanks were amplified by PCR using genomic DNA of CEN.PK113-7D as template which was isolated as described by Lõoke et al., 2011. The LF was amplified using primerset SEQ ID NO: 51 and SEQ ID NO: 52, resulting in a 577 bp fragment (SEQ ID NO: 53). The RF was amplified using primer set SEQ ID NO: 54 and SEQ ID NO: 55, resulting in a 581 bp fragment (SEQ ID NO: 56).

PCR reactions were performed using PrimeStar GXL DNA polymerase (Takara / Catno. R050A) according to supplier's instructions and a PCR program known to a person skilled in the art. The LF and RF PCR fragments were purified using the NucleoSpin Gel and PCR Clean-up kit (Machery-Nagel, distributed by Bioké, Leiden, the Netherlands) according to manufacturer's instructions.

By integration of the YFP cassette a stretch of 1 kbp genomic DNA is knocked out at the INT1 locus.

### Double-stranded DNA (ds-DNA) donor YFP expression cassette with 50 bp connector flanks

A double-stranded donor DNA cassette coding for the Yellow Fluorescent Protein (YFP) variant Venus (Nagai *et al.,* 2002), was prepared via a Golden-Gate assembly reaction of individual promoter (P), orf (O) and terminator (T) sequences in an appropriate E. *coli* vector. The assembled POT cassette was amplified via a PCR reaction with primers indicated in SEQ ID NO: 57 and SEQ ID NO: 58. In a second PCR, 50 bp connector sequences are added using primer sets indicated in SEQ ID NO: 59 and SEQ ID NO: 60. This resulted in an YFP expression cassette that included 50 bp connector sequences at the 5' and 3' ends of the expression cassette (SEQ ID NO: 50). The Q5 DNA polymerase (part of the Q5^{®} High-Fidelity 2X Master Mix, New England Biolabs, supplied by Bioké, Leiden, the Netherlands. Cat no. M0492S) was used in the PCR reaction, which was performed according to manufacturer's instructions. The PCR fragment was purified using the NucleoSpin Gel and PCR Clean-up kit (Machery-Nagel, distributed by Bioké, Leiden, the Netherlands) according to manufacturer's instructions.

### DNA concentrations

All DNA concentrations, flanks (LF and RF), including the guide RNA expression cassette PCR fragment and pRN1120, were determined using a NanoDrop device (ThermoFisher, Life Technologies, Bleiswijk, the Netherlands), providing the concentrations in nanogram per microliter. Based on these measurements, an amount of 1 µg T7 controlled guide RNA fragment, 100 ng EcoRI/Xhol restricted pRN1120, 100 ng YFP donor expression cassette and 2x 100 ng flanks (long) or 4x50 ng flanks (100 bp flanks) were used in the transformation experiments.

### Yeast transformation

Strains CSN007 and CSN008 which are pre-expressing CAS9 and T7 RNAP, were inoculated in YPD-G418 medium (10 grams per liter of yeast extract, 20 grams per liter of peptone, 20 grams per liter of dextrose, 200 µg G418 (Sigma Aldrich, Zwijndrecht, the Netherlands) per ml. Subsequently, strains CSN007 and CSN008 were transformed with 1 µg T7 guide RNA cassette, 100 ng *Xhol*/*EcoRI* restricted pRN1120, 100 ng of each flank (LF and RF) and 100 ng of YFP expression cassette donor as indicated in Table 2, using the LiAc/SS carrier DNA/PEG method (Gietz and Woods, 2002). All transformations were performed in triplicate with exception of the controls which were done as single measurement. The transformation mixtures were plated on YPD-agar (10 grams per liter of yeast extract, 20 grams per liter of peptone, 20 grams per liter of dextrose, 20 grams per liter of agar) containing 200 µg nourseothricin (NTC, Jena Bioscience, Germany) and 200 µg G418 (Sigma Aldrich, Zwijndrecht, the Netherlands) per ml. The plates were incubated at 30 degrees Celsius until colonies appeared on the plates.

**Table 2. Overview of T7 controlled guide RNA's used in transformation**

| **Transfer mation** | **T7 promoter strength** | **sgRNA sequence** | **Strain** | **Con5-YFP-Con3** | **FLANKS 100 bp (4x50 ng)** | **FLANKS 500bp (2x100 ng)** | **pRN1120 x XhoI/EcoRI (100 ng)** |
|---|---|---|---|---|---|---|---|
| #1 | Strong | SEQ ID NO: 36 | CSN007 | X | X | | X |
| #2 | Medium | SEQ ID NO: 37 | CSN007 | X | X | | X |
| #3 | Weak | SEQ ID NO: 38 | CSN007 | X | X | | X |
| #4 | Wild type | SEQ ID NO: 39 | CSN007 | X | X | | X |
| #5 | Strong | SEQ ID NO: 36 | CSN007 | X | | X | X |
| #6 | Medium | SEQ ID NO: 37 | CSN007 | X | | X | X |
| #7 | Weak | SEQ ID NO: 38 | CSN007 | X | | X | X |
| #8 | Wild type | SEQ ID NO: 39 | CSN007 | X | | X | X |
| #9 | Strong | SEQ ID NO: 36 | CSN008 | X | X | | X |
| #10 | Medium | SEQ ID NO: 37 | CSN008 | X | X | | X |
| #11 | Weak | SEQ ID NO: 38 | CSN008 | X | X | | X |
| #12 | Wild type | SEQ ID NO: 39 | CSN008 | X | X | | X |
| #13 | Strong | SEQ ID NO: 36 | CSN008 | X | | X | X |
| #14 | Medium | SEQ ID NO: 37 | CSN008 | X | | X | X |
| #15 | Weak | SEQ ID NO: 38 | CSN008 | X | | X | X |
| #16 | Wild type | SEQ ID NO: 39 | CSN008 | X | | X | X |
| #17 | | | CSN007 | X | X | | X |
| #18 | | | CSN007 | X | | X | X |
| #19 | | | CSN008 | X | X | | X |
| #20 | | | CSN008 | X | | X | X |

### Results

The transformation experiment outlined above in Table 2 was performed and after transformation, the cells were placed on YPD selective plates. To confirm integration of the YFP expression cassette donor at the INT1, transformants of each transformation were analyzed for YFP fluorescence on the Qpix450.

**Table 3. Overview of analysis of transformants by Qpix450**

| **Transformation** | **T7 promoter strength** | **Level of T7 RNAP expression** | **Length of Flanks** | **Total number of transformants** | **Number of fluorescent transformants** | **Success rate %** |
|---|---|---|---|---|---|---|
| #1 | Strong | High | 100 bp | 66 | 1 | 1.5% |
| #2 | Medium | High | 100 bp | 38 | 0 | 0% |
| #3 | Weak | High | 100 bp | 150 | 1 | 0.6% |
| #4 | Wild type | High | 100 bp | 69 | 16 | 23% |
| #5 | Strong | High | 500 bp | 56 | 5 | 9% |
| #6 | Medium | High | 500 bp | 29 | 0 | 0% |
| #7 | Weak | High | 500 bp | 96 | 3 | 3% |
| #8 | Wild type | High | 500 bp | 115 | 52 | 45% |
| #9 | Strong | Medium | 100 bp | 81 | 3 | 4% |
| #10 | Medium | Medium | 100 bp | 75 | 0 | 0% |
| #11 | Weak | Medium | 100 bp | 250 | 4 | 1.6% |
| #12 | Wild type | Medium | 100 bp | 200 | 20 | 10% |
| #13 | Strong | Medium | 500 bp | 99 | 10 | 10% |
| #14 | Medium | Medium | 500 bp | 7 | 0 | 0% |
| #15 | Weak | Medium | 500 bp | 250 | 10 | 4% |
| #16 | Wild type | Medium | 500 bp | 135 | 44 | 33% |
| #17 | | High | 100 bp | 24 | 0 | 0% |
| #18 | | High | 500 bp | 15 | 2 | 13% |
| #19 | | Medium | 100 bp | 68 | 2 | 3% |
| #20 | | Medium | 500 bp | 48 | 4 | 8% |

Results of the transformation demonstrate that the integration of the YFP expression donor cassette is enhanced by the CRISPR Cas system when guide RNA is expressed under control of the T7 promoter. The wildtype T7 promoter being the most effective out of the set of T7 promoters that was tested, here with a maximum editing frequency of 45% for the integration of a YFP cassette. Higher levels of integration of the YFP cassette are obtained when T7 RNAP is expressed at a higher level (under control of Ptdh3, strain CSN007). Above results demonstrate the functionality of the T7 system for guide RNA expression in S. *cerevisiae.*

### Example 5: Expression of sgRNA in Aspergillus niger using a sgRNA expression cassette with a T7 promoter part A

This example describes the disruption of the *fnwA* locus in genomic DNA of A. *niger* using CAS9 in combination with a T7 promoter in front of a single-guide RNA (sgRNA) expression cassette and donor DNA. Strains with a mutation in the *fwnA* gene will have a color change in the spores from black to fawn (Jørgensen et al., 2011).

### Donor DNA

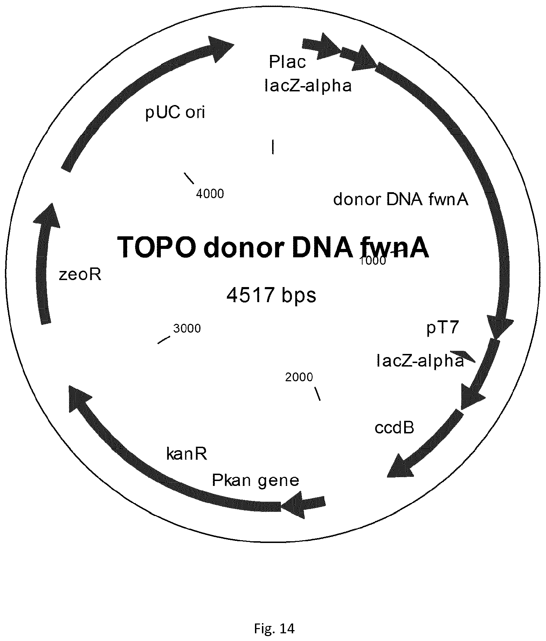

A gBlock fragment was synthesized at IDT (gBlocks^{®} Gene Fragments, Integrated DNA Technologies, Inc) that contained the donor DNA for the desired mutation (SEQ ID NO: 62). This DNA was cloned into a TOPO Zero Blunt vector with the Zero Blunt TOPO PCR Cloning Kit of Invitrogen and named TOPO donor DNA fwnA (SEQ ID NO: 63). A plasmid map of TOPO donor DNA fwnA is depicted in Figure 14. PCR amplification of the donor DNA from the TOPO-vector was done with Phusion DNA polymerase (New England Biolabs) using forward primer as set out in SEQ ID NO: 64 and reverse primer as set out in SEQ ID NO: 65 according to standard PCR protocols. The PCR fragments were purified with the PCR purification kit from Macherey Nagel according to manufacturer's instructions. DNA concentrations were measured using the NanoDrop (ND-1000 Spectrophotometer, Thermo Scientific).

### Construction of BG-AMA17 plasmid

PCR amplification of the Cas9 expression cassette (construction of BG-C20 Cas9 expression cassette is described in WO2016110453A1) was performed using Phusion DNA polymerase (New England Biolabs), and forward primer as set out in SEQ ID NO: 66 and reverse primer as set out in SEQ ID NO: 67. Both primers contained flanks with a Kpnl restriction site. The PCR products were purified with a PCR purification kit from Macherey Nagel (distributed by Bioké, Leiden, the Netherlands) according to manufacturer's instructions. The DNA concentration was measured using a NanoDrop (ND-1000 Spectrophotometer, Thermo Fisher Scientific).

Backbone vector BG-AMA8 (described in WO2016110453A1) and the obtained Kpnl flanked PCR fragment of the Cas9 expression cassette were digested with Kpnl (NEB-enzymes) and purified with a PCR purification kit from Macherey Nagel (distributed by Bioké, Leiden, The Netherlands). Digested BG-AMA8 backbone vector and Cas9 cassette PCR product were ligated with T4 ligation

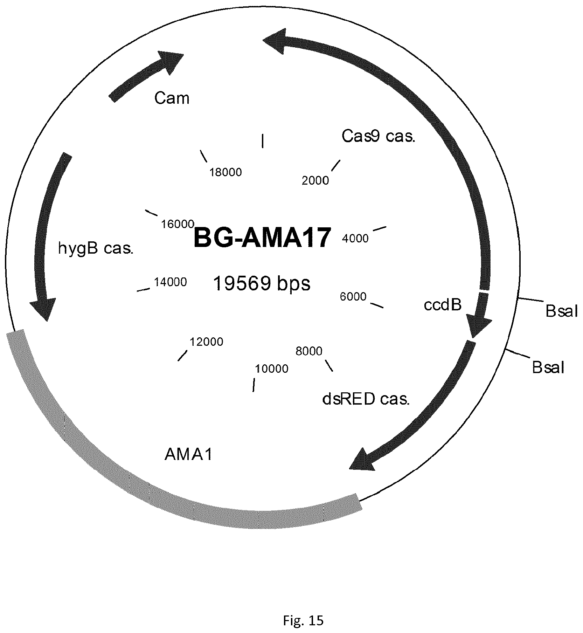

(Invitrogen) according to manufacturer's instructions. The ligation mix was transformed to ccdB resistant E. coli cells (Invitrogen) according to manufacturer's instructions. Several clones were checked with restriction enzyme analysis and a done having the correct restriction pattern was named BG-AMA17 (SEQ ID NO: 68). A plasmid map of BG-AMA17 is provided in Figure 15. Plasmid BG-AMA17 contains a Cas9 expression cassette expressed from a promoter and terminator, a dsRED cassette and a HygB marker for selection in A. *niger.*

### Construction of plasmid BG-AMA18 and BG-AMA19

gBlock fragments were synthesized at IDT (gBlocks^{®} Gene Fragments, Integrated DNA Technologies, Inc) that contained the T7 sgRNA expression cassette targeting fwnA with a T7 wt promoter (SEQ ID NO: 69) and with a T7 strong promoter (SEQ ID NO: 70). These DNA fragments were cloned into a TOPO Zero Blunt vector with the Zero Blunt TOPO PCR Cloning Kit of Invitrogen.

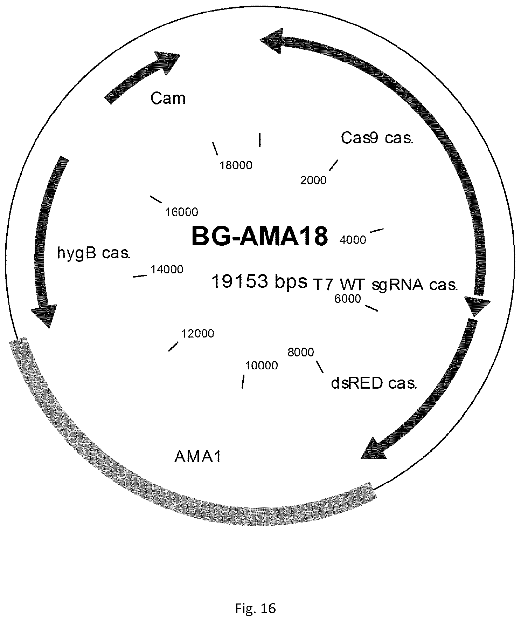

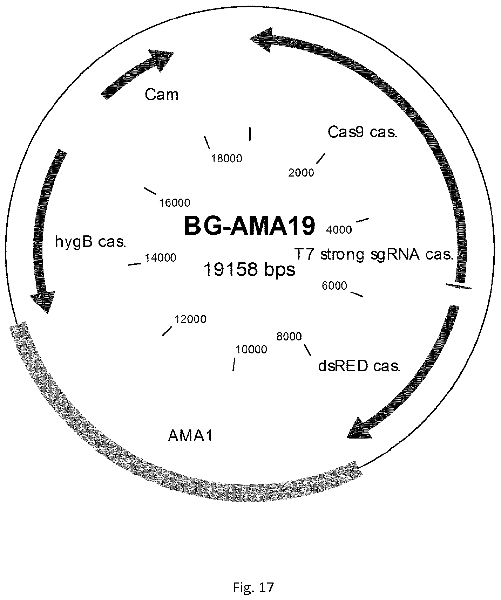

The constructed TOPO DNA vectors were cloned using a Golden Gate reaction (according to example 1 in patent application WO2013/144257) into the receiving backbone vector BG-AMA17. This resulted in the vector named BG-AMA18 → with T7 wt promoter (SEQ ID NO: 71) and the vector named BG-AMA19 → with T7 strong promoter (SEQ ID NO: 72). The BG-AMA18 and BG-AMA19 vectors were checked by E. coli colony PCR to check the size of the cloned sgRNA fwnA cassette. The PCR was performed using Phusion polymerase (New England Biolabs) according to standard PCR protocols using forward primer as set out in SEQ ID NO: 73 and reverse primer as set out in SEQ ID NO: 74. Plasmid maps of BG-AMA18 and BG-AMA19 are depicted in Figure 16 and Figure 17.

### Strain

In this example *Aspergillus niger* strain GBA 302 (ΔgIaA, ΔpepA, ΔhdfA) was used in the transformation experiments. The construction of GBA 302 is described in patent application WO2011/009700.

### Transformation

Protoplast transformation was performed as described in patent applications WO1999/32617 and WO1998/46772, except for the use of ATA (Aurintricarboxylic acid = nuclease inhibitor) in the transformation mixture.

### AMA-vectors used in the transformations (1.5 µg/transformation):

1. AMA-vector BG-AMA17 (SEQ ID NO: 68; Figure 15) → AMA hygB/Cas9
2. AMA-vector BG-AMA18 (SEQ ID NO: 71; Figure 16) → AMA hygB/Cas9/T7 WT sgRNA cassette
3. AMA-vector BG-AMA19 (SEQ ID NO: 72; Figure 17) → AMA hygB/Cas9/ T7 strong sgRNA cassette

Table 4 shows the specific amounts of DNA transformed to the strain GBA 302 in each separate transformation.

**Table 4: Overview of performed transformations. Transformations were performed to strain GBA 302. Different AMA plasmids were used as circular plasmids. As donor DNA, a PCR fragment containing desired mutations was included in some of the transformations, as indicated in the table below. In total 6 transformations were performed.**

| **Transfor mation** | **Strain** | **AMA plasmid (Cas9)** | | **Donor DNA** |
|---|---|---|---|---|
| | | **Concentration / name** | **sgRNA promoter** | |
| 1 | GBA 302 | 1.5 µg BG-AMA17 | X | 0 µg |
| 2 | GBA 302 | 1.5 µg BG-AMA17 | X | 2 µg PCR-fragment |
| 3 | GBA 302 | 1.5 µg BG-AMA18 | T7 wt | 0 µg |
| 4 | GBA 302 | 1.5 µg BG-AMA19 | T7 strong | 0 µg |
| 5 | GBA 302 | 1.5 µg BG-AMA18 | T7 wt | 2 µg PCR-fragment |
| 6 | GBA 302 | 1.5 µg BG-AMA19 | T7 strong | 2 µg PCR-fragment |

After transformation, the protoplasts were plated on regeneration media plates containing 60 µg/ml Hygromycin B (Invitrogen). All plates were incubated at 30 °C for 4-6 days.

Results of the transformation can be found in table 5.

**Table 5: Results of the 6 transformation experiments with the number of transformants containing the fwnA phenotype / the total number of transformants obtained and the percentage of fawn colored colonies, containing the fwnA phenotype, identified in the total number of transformants.**

| **Transfer mation** | **AMA plasmid (Cas9)** | | **Donor DNA** | **No. of fwnA phenotype / total no. transformants** | **% of fwnA phenotype of total no. transformants** |
|---|---|---|---|---|---|
| | **Concentration / name** | **sgRNA promoter** | | | |
| 1 | 1.5 µg BG-AMA17 | X | 0 µg | 0/77 | 0 |
| 2 | 1.5 µg BG-AMA17 | X | 2 µg PCR-fragment | 0/101 | 0 |
| 3 | 1.5 µg BG-AMA18 | T7 wt | 0 µg | 0/52 | 0 |
| 4 | 1.5 µg BG-AMA19 | T7 strong | 0 µg | 0/65 | 0 |
| 5 | 1.5 µg BG-AMA18 | T7 wt | 2 µg PCR-fragment | 6/45 | 13 |
| 6 | 1.5 µg BG-AMA19 | T7 strong | 2 µg PCR-fragment | 10/98 | 10 |

The transformants from all transformation plates were counted and scored for the fawn spore phenotype characteristic of the fwnA mutation.

In all transformations without donor DNA (transformation no. 1, 3 and 4) no fawn colored transformants were obtained. In transformation 2 without sgRNA cassette also no fawn colored transformants were obtained.

In transformation 5 and 6 (GBA 302, Cas9, sgRNA → WT or strong T7 promoter in front of sgRNA and with donor DNA) 13 - 10% of the transformants had a fwnA phenotype.

When comparing transformations 2 with 5 and 6 the only difference is the presence of the T7 sgRNA cassette in the AMA-plasmid. It seems that A. *niger* is able to induce the T7 promoter in front of the sgRNA which together with the Cas9 and donor DNA causes the fawn coloring of some transformants (13 - 10% of the transformants).

When comparing transformation 5 (T7 wt sgRNA) with 6 (T7 strong sgRNA) an equal percentage of fawn coloring was obtained.

### Colony PCR to produce DNA fragments for sequencing

Spores of transformations 5 and 6 were plated on a PDA plate (Difco) and incubated for 2-3 days at 30 °C in an incubator. For each tested colony a sample of the colony was taken with an inoculation loop and put in 25 µl Glucanex^{™} solution (50 mg/ml Glucanex^{™} dissolved in KC buffer (60 g/l KCI, 2 g/l Citric acid, adjusted with KOH/HCI to pH 6.2)) in an Eppendorf cup. After 1 hour incubation at 37°C, 75 µl DNA dilution buffer was added to each cup followed by boiling for 5 minutes in PCR apparatus with heated lid. After boiling 100 µl milIQ water was added and mixed very mildly by pipetting up and down three times. Subsequently, 5 µl chromosomal DNA template was pipetted carefully from the top of the solution and added in the PCR-mix for each reaction (without taking along cell debris from the bottom). The PCR reactions were performed according to standard PCR protocols using Phusion polymerase (New England Biolabs) amplifying the genomic fwnA6 location by using the forward primer as set out in SEQ ID NO: 75 and reverse primer as set out in SEQ ID NO: 76. The PCR fragments were purified with the PCR purification kit from Macherey Nagel according to the manual.

### Confirming the genomic mutation in fwnA by sequencing

PCR for sequencing was done with BigDye Terminator v3.1 Cycle Sequencing kit of Applied Biosystems according to the manual by using the forward primer as set out in SEQ ID NO: 75 and purified colony PCR-fragments as template. Sequencing PCR product was cleaned by ethanol/EDTA precipitation according to supplier manual. The fwnA6 sequence PCR fragment pellet was dissolved in 10 µl HiDi Formamide of Applied Biosystems and suspension used for sequence analysis with the 3500 Genetic Analyzer of Applied Biosystems (Sanger sequencer). For each transformation, a maximum of 10 transformants showing a fwnA phenotype were sequenced.

**Table 6: Results of the sequencing indicated as the percentage of transformants that contain the designed 5 bp deletion of total no. fwnA phenotype transformants and the percentage of designed 5 bp deletion of the total number of transformants.**

| **Transfor mation** | **AMA plasmid (Cas9)** | | **Donor DNA** | **% of 5 bp designed deletion of total no. fwnA phenotype transformants** | **% of 5 bp designed deletion of total no. transformants** |
|---|---|---|---|---|---|
| | **Name** | **sgRNA promoter** | | | |
| 5 | BG-AMA18 | WT T7 | 2 µg PCR-fragment | 100 | 13 |
| 6 | BG-AMA19 | Strong T7 | 2 µg PCR-fragment | 100 | 10 |

All sequenced fawn colored transformants had the designed 5 bp deletion in the fwnA gene.

### Example number 6: Expression of sgRNA in Aspergillus niger using a sgRNA expression cassette with a T7 promoter in combination with T7 RNAP protein

This example describes the disruption of the *fnwA* locus in genomic DNA of A. *niger* using CAS9 in combination with a T7 promoter in front of a single-guide RNA (sgRNA) expression cassette, T7 RNAP protein and donor DNA. Strains with a mutation in the *fwnA* gene will have a color change in the spores from black to fawn (Jørgensen et al., 2011).

### Donor DNA

Same as example 5

### Construction BG-AMA17

Same as example 5

### Construction of plasmid BG-AMA18 and BG-AMA19

Same as example 5

### Strain

Same as example 5

### Transformation

Protoplast transformation was performed as described in patent applications WO1999/32617 and WO1998/46772, except for the use of ATA (Aurintricarboxylic acid = nuclease inhibitor) in the transformation mixture. In some of the transformations T7 RNAP protein was used (New England BioLabs).

### AMA-vectors used in the transformations (1.5 µg/transformation):

4. AMA-vector BG-AMA17 (SEQ ID NO: 68; Figure 15) → AMA hygB/Cas9
5. AMA-vector BG-AMA18 (SEQ ID NO: 71; Figure 16) → AMA hygB/Cas9/T7 WT sgRNA cassette
6. AMA-vector BG-AMA19 (SEQ ID NO: 72; Figure 17) → AMA hygB/Cas9/ T7 strong sgRNA cassette

Table 7 shows the specific amounts of DNA/protein transformed to the strain GBA 302 in each separate transformation.

**Table 7: Overview of performed transformations. Transformations were performed to strain GBA 302. Different AMA plasmids were used as circular plasmids and in some cases T7 RNAP protein. As donor DNA, a PCR fragment containing desired mutations was included in some of the transformations, as indicated in the table below. In total 8 transformations were performed.**

| **Transfer mation** | **Strain** | **AMA plasmid (with Cas9)** | | **T7 RNAP protein** | **Donor DNA** |
|---|---|---|---|---|---|
| | | **Concentration/name** | **sgRNA** | | |
| 1 | GBA 302 | 1.5 µg BG-AMA17 | X | X | 0 µg |
| 2 | GBA 302 | 1.5 µg BG-AMA17 | X | X | 2 µg PCR-fragment |
| 3 | GBA 302 | 1.5 µg BG-AMA18 | T7 wt promoter | X | 2 µg PCR-fragment |
| 4 | GBA 302 | 1.5 µg BG-AMA19 | T7 strong promoter | X | 2 µg PCR-fragment |
| 5 | GBA 302 | 1.5 µg BG-AMA18 | T7 wt promoter | 100U | 0 µg |
| 6 | GBA 302 | 1.5 µg BG-AMA19 | T7 strong promoter | 100U | 0 µg |
| 7 | GBA 302 | 1.5 µg BG-AMA18 | T7 wt promoter | 100U | 2 µg PCR-fragment |
| 8 | GBA 302 | 1.5 µg BG-AMA19 | T7 strong promoter | 100U | 2 µg PCR-fragment |

After transformation the protoplasts were plated on regeneration media plates containing 60 µg/ml Hygromycin B (Invitrogen). All plates were incubated at 30 °C for 4-6 days.

Results of the transformation can be found in table 8.

**Table 8: Results of the 8 transformation experiments with the number of transformants containing the fwnA phenotype / the total number of transformants obtained and the percentage of fawn colored colonies, containing the fwnA phenotype, identified in the total number of transformants.**

| **Transfer mation** | **AMA plasmid (with Cas9)** | | **T7 RNAP protein** | **Donor DNA** | **No. of fwnA phenotype / total no. transformants** | **% of fwnA phenotype of total no. transformants** |
|---|---|---|---|---|---|---|
| | **Name** | **sgRNA promoter** | | | | |
| 1 | BG-AMA17 | X | X | 0 µg | 0/∼400 | 0 |
| 2 | BG-AMA17 | X | X | 2 µg PCR-fragment | 0/∼600 | 0 |
| 3 | BG-AMA18 | T7 wt | X | 2 µg PCR-fragment | 16/∼400 | 4 |
| 4 | BG-AMA19 | T7 strong | X | 2 µg PCR-fragment | 14/∼400 | 4 |
| 5 | BG-AMA18 | T7 wt | 100U | 0 µg | 0/∼350 | 0 |
| 6 | BG-AMA19 | T7 strong | 100U | 0 µg | 0/∼300 | 0 |
| 7 | BG-AMA18 | T7 wt | 100U | 2 µg PCR-fragment | 47/∼600 | 8 |
| 8 | BG-AMA19 | T7 strong | 100U | 2 µg PCR-fragment | 37/∼600 | 6 |

The transformants from all transformation plates were counted and scored for the fawn spore phenotype characteristic of the fwnA mutation.

In all transformations without donor DNA (transformation no. 1, 5 and 6) no fawn colored transformants were obtained. In transformation 2 without guide RNA also no fawn colored transformants were obtained.

In transformation 3 and 4 (GBA 302, Cas9, sgRNA → WT or strong T7 promoter in front of sgRNA and with donor DNA) 4% of the transformants had a fwnA phenotype. In transformation 7 and 8 (GBA 302, Cas9, sgRNA (WT or strong T7 promoter in front of sgRNA, T7 RNAP protein and with donor DNA) 8 - 6% of the transformants had a fwnA phenotype.

When comparing transformations 2 with 3 (both controls without sgRNA) and 4, the only difference is the presence of the T7 sgRNA cassette in the AMA-plasmid. It seems that A. *niger* is able to use the T7 promoter in front of the sgRNA and express functional guide RNA. Together with the Cas9 and donor DNA, the system causes the fawn coloring of some transformants (4% of the transformants).

This demonstrates that a T7 promoter in front of sgRNA can be successfully used in A. *niger* for genome editing. When comparing transformations 3+4 with 7+8, the results show that adding T7 RNAP protein in the transformation increases the percentage of fawn colored transformants. Thus, by adding T7 RNAP protein the CRISPR/Cas mediated fwn mutation efficiency increases.

### Colony PCR SDS/LiAC to produce DNA fragment for sequencing

Spores of transformations 3, 4, 7 and 8 were plated on a PDA plate (Difco) and incubated for 2-3 days at 30 °C in an incubator. For each tested colony a sample of the colony was taken with an inoculation loop and put in 25 µl Glucanex^{™} solution (50 mg/ml Glucanex^{™} dissolved in KC buffer (60 g/l KCI, 2 g/l Citric acid, adjusted with KOH/HCI to pH 6.2)) in an Eppendorf cup. After 1 hour incubation at 37°C, 75 µl DNA dilution buffer was added to each cup followed by boiling for 5 minutes in PCR apparatus with heated lid. After boiling 100 µl millQ water was added and mixed very mildly by pipetting up and down three times. Subsequently, 5 µl chromosomal DNA template was pipetted carefully from the top of the solution and added in the PCR-mix for each reaction (without taking along cell debris from the bottom). The PCR reactions were performed according to standard PCR protocols using Phusion polymerase (New England Biolabs) amplifying the genomic fwnA6 location by using the forward primer as set out in SEQ ID NO: 75 and reverse primer as set out in SEQ ID NO: 76. The PCR fragments were purified with the PCR purification kit from Macherey Nagel according to the manual.

### Confirming the genomic mutation in fwnA by sequencing

PCR for sequencing was done with BigDye Terminator v3.1 Cycle Sequencing kit of Applied Biosystems according to the manual by using the forward primer as set out in SEQ ID NO: 71 and purified colony PCR-fragments as template. Sequencing PCR product was cleaned by ethanol/EDTA precipitation according to supplier manual. The fwnA6 sequence PCR fragment pellet was dissolved in 10 µl HiDi Formamide of Applied Biosystems and suspension used for sequence analysis with the 3500 Genetic Analyzer of Applied Biosystems (Sanger sequencer). For each transformation, a maximum of 16 transformants showing a fwnA phenotype were sequenced.

**Table 9: Results of the sequencing indicated as the percentage of transformants that contain the designed 5 bp deletion of total no. fwnA phenotype transformants and the percentage of designed 5 bp deletion of the total number of transformants.**

| **Transfer mation** | **AMA plasmid (Cas9)** | | **T7 RNAP protein** | **Donor DNA** | **% of 5 bp designed deletion of total no. fwnA phenotype transformants** | **% of 5 bp designed deletion of total no. transformants** |
|---|---|---|---|---|---|---|
| | **Name** | **sgRNA promoter** | | | | |
| 3 | BG-AMA18 | T7 wt | X | 2 µg PCR-fragment | 100 | 4 |
| 4 | BG-AMA19 | T7 strong | X | 2 µg PCR-fragment | 100 | 4 |
| 7 | BG-AMA18 | T7 wt | 100U | 2 µg PCR-fragment | 100 | 8 |
| 8 | BG-AMA19 | T7 strong | 100U | 2 µg PCR-fragment | 100 | 6 |

All sequenced fawn colored transformants had the designed 5 bp deletion in the fwnA gene.

### References

1. Qi et al., "Repurposing CRISPR as an RNA-Guided Platform for Sequence-Specific Control of Gene Expression," Cell 152, no. 5 (February 28, 2013): 1173-83, doi:10.1016/j.cell.2013.02.022.
2. van Dijken et al., "An interlaboratory comparison of physiological and genetic properties of four Saccharomyces cerevisiae strains," Enzyme Microb Technol. 2000 Jun 1;26(9-10):706-714.
3. Gilbert et al., "CRISPR-Mediated Modular RNA-Guided Regulation of Transcription in Eukaryotes," Cell 154, no. 2 (July 18, 2013): 442-51, doi:10.1016/j.cell.2013.06.044.
4. Kim et al., "High Cleavage Efficiency of a 2A Peptide Derived from Porcine Teschovirus-1 in Human Cell Lines, Zebrafish and Mice," PLOS ONE 6, no. 4 (April 29, 2011): e18556, doi:10.1371/journal.pone.0018556.
6. Szafraniec et al., "Trans-Acting Antigenomic HDV Ribozyme for Production of in Vitro Transcripts with Homogenous 3' Ends," Methods in Molecular Biology (Clifton, N.J.) 941 (2012): 99-111, doi:10.1007/978-1-62703-113-4_8.
7. Orr-Weaver et al., "Genetic applications of yeast transformation with linear and gapped plasmids, " Methods Enzymol. 1983;101:228-45.
8. Lõoke et al. Biotechniques. 2011 May;50(5):325-8. Extraction of genomic DNA from yeasts for PCR-based applications.
9. Gietz and Woods., "Transformation of yeast by lithium acetate/single-stranded carrier DNA/polyethylene glycol method," Methods Enzymol. 2002;350:87-96.
10. Sikorski and Hieter. "A system of shuttle vectors and yeast host strains designed for efficient manipulation of DNA in Saccharomyces cerevisiae," Genetics. 1989 May;122(1):19-27.
11. Jones, J. Andrew et al., "ePathOptimize: A Combinatorial Approach for Transcriptional Balancing of Metabolic Pathways," Scientific Reports. 2015 5 June: 11301-6.
12. Temme, Karsetn et al., "Modular Control of Multiple Pathways Using Engineered Orthogonal T7 Polymerases. Nucleic Acids Research 2012 40(17): 8773-8781.
13. Gao Y and Zhao Y. J Integr Plant Biol. 2014 Apr;56(4):343-9. Self-processing of ribozyme-flanked RNAs into guide RNAs in vitro and in vivo for CRISPR-mediated genome editing.
14. Chu et al. Nat Biotechnol. 2015 May;33(5):543-8.
15. Maruyana et al. Nat Biotechnol. 2015 May ; 33(5): 538-542.
16. Song et al. Nature communications | doi: 10.1038/ncomms10548
17. Yu et al. Cell Stem Cell. 2015 February 5; 16(2): 142-147.
18. Tycko J, Myer VE, Hsu PD. Methods for Optimizing CRISPR-Cas9 Genome Editing Specificity. Mol Cell. 2016 Aug 4;63(3):355-70.
19. Nelson CE, Gersbach CA. Cas9 loosens its grip on off-target sites. Nat Biotechnol. 2016 Mar;34(3):298-9.
20. Benton et al., Molecular and cellular biology, Jan. 1990, p. 353-360.
21. Shis et al., Molecular Systems Biology 10: 745 | 2014
22. Romanienko et al. PLOS ONE | DOI:10.1371/journal.pone.0148362 February 5, 2016.
23. Hsu PD, Lander ES, Zhang F. Cell. 2014 Jun 5;157(6):1262-78. Development and applications of CRISPR-Cas9 for genome engineering.
24. Sander JD, Joung JK. Nat Biotechnol. 2014 Apr;32(4):347-55. doi: 10.1038/nbt.2842. Epub 2014 Mar 2. CRISPR-Cas systems for editing, regulating and targeting genomes.
25. Zetsche B, Gootenberg JS, Abudayyeh OO, Slaymaker IM, Makarova KS, Essletzbichler P, Volz SE, Joung J, van der Oost J, Regev A, Koonin EV, Zhang F. Cpf1 is a single RNA-guided endonuclease of a class 2 CRISPR-Cas system. Cell. 2015 Oct 22;163(3):759-71. doi: 10.1016/j.cell.2015.09.038. Epub 2015 Sep 25.
26. Didovyk A, Borek B, Tsimring L, Hasty J. Transcriptional regulation with CRISPR-Cas9: principles, advances, and applications. Curr Opin Biotechnol. 2016 Aug;40:177-84. doi: 10.1016/j.copbio.2016.06.003. Epub 2016 Jun 23.
27. Young and Dong, (2004), Nucleic Acids Research 32, (7) electronic access http://nar.oupjournals.org/cgi/reprint/32/7/e59 or Gupta et al. (1968), Proc. Natl. Acad. Sci USA, 60: 1338-1344; Scarpulla et al. (1982), Anal. Biochem. 121: 356-365; Stemmer et al. (1995), Gene 164: 49-53.
28. Ho SN, Hunt HD, Horton RM, Pullen JK, Pease LR "Site-directed mutagenesis by overlap extension using the polymerase chain reaction"
29. Molecular Biology: Current Innovations and Future Trends. (Eds. A.M. Griffin and H.G.Griffin. ISBN 1-898486-01-8; 1995 Horizon Scientific Press, PO Box 1, Wymondham, Norfolk, U.K.
30. Jørgensen TR, Park J, Arentshorst M, van Welzen AM, Lamers G, Vankuyk PA, Damveld RA, van den Hondel CA, Nielsen KF, Frisvad JC, Ram AF. Fungal Genet Biol. 2011 May;48(5):544-53. The molecular and genetic basis of conidial pigmentation in Aspergillus niger.
31. DiCarlo JE, Norville JE, Mali P, Rios X, Aach J, Church GM. Genome engineering in Saccharomyces cerevisiae using CRISPR-Cas systems. Nucleic Acids Res. 2013;41(7):4336-4

## Claims

1. Use of a viral single-subunit DNA-dependent RNA polymerase selected from a T3, SP6, K11 or T7 RNA polymerase, for the expression within a fungal cell of a guide-RNA for an RNA-guided nuclease system, wherein the guide-RNA is encoded by a polynucleotide that is operably linked to a viral single-subunit DNA-dependent RNA polymerase promoter selected from a T3, SP6, K11 or T7 RNA polymerase promoter and wherein the fungal cell is a yeast cell or a filamentous fungus cell.

2. Use according to claim 1, wherein the fungal cell is a a yeast cell selected from the group consisting of *Candida, Hansenula, Issatchenkia, Kluyveromyces, Pichia, Saccharomyces, Schizosaccharomyces, Yarrowia and Zygosaccharomyces;* preferably a filamentous fungus cell that is from a genus selected from the group consisting of *Acremonium, Aspergillus, Chrysosporium, Myceliophthora, Penicillium, Talaromyces, Rasamsonia, Thielavia, Fusarium* and *Trichoderma.*

3. Use according to claim 1 or 2, wherein the guide-RNA is expressed from a linear nucleic acid construct, from a genome or from a vector, preferably a plasmid.

4. Use according to any one of claims 1 - 3, wherein multiple, distinct guide-RNA's are expressed from either a sole single-subunit DNA-dependent RNA polymerase promoter selected from a T3, SP6, K11 or T7 RNA polymerase promoter or from multiple single-subunit DNA-dependent RNA polymerase promoters selected from a T3, SP6, K11 or T7 RNA polymerase promoter.

5. Use according to any one of claims 1 - 4, wherein the guide-RNA is expressed from one or more single-subunit DNA-dependent RNA polymerase promoters from a library of single-subunit DNA-dependent RNA polymerase promoters selected from a T3, SP6, K11 or T7 RNA polymerase promoter.

6. Use according to any one of claims 1 - 5, wherein the cell is deficient in an NHEJ (non-homologous end joining) component.

7. A method for expression within a fungal cell of a guide-RNA for an RNA-guided nuclease system, wherein the guide-RNA is encoded by a polynucleotide that is operably linked to a viral single-subunit DNA-dependent RNA polymerase promoter selected from a T3, SP6, K11 or T7 RNA polymerase promoter, wherein transcription of the guide-RNA is performed by a viral single-subunit DNA-dependent RNA polymerase selected from aT3, SP6, K11 or T7 RNA polymerase, and wherein the fungal cell is a yeast cell or a filamentous fungus cell.

8. A method according to claim 7, wherein the fungal cell is a a yeast cell selected from the group consisting of *Candida, Hansenula, Issatchenkia, Kluyveromyces, Pichia, Saccharomyces, Schizosaccharomyces, Yarrowia and Zygosaccharomyces;* preferably a filamentous fungus cell that is from a genus selected from the group consisting of *Acremonium, Aspergillus, Chrysosporium, Myceliophthora, Penicillium, Talaromyces, Rasamsonia, Thielavia, Fusarium* and *Trichoderma*

9. The method according to any one of claims 7-8, wherein multiple, distinct guide-RNA's are expressed from either a sole single-subunit DNA-dependent RNA polymerase promoter selected from a T3, SP6, K11 or T7 RNA polymerase promoter or from multiple single-subunit DNA-dependent RNA polymerase promoters selected from a T3, SP6, K11 or T7 RNA polymerase promoter.

10. The method according to any one of claims 7-9, wherein the guide-RNA is expressed from one or more single-subunit DNA-dependent RNA polymerase promoters from a library of single-subunit DNA-dependent RNA polymerase promoters selected from a T3, SP6, K11 or T7 RNA polymerase promoter.

11. The method according to any one of claims 7 to 10, wherein the cell is deficient in an NHEJ (non-homologous end joining) component.

12. A composition comprising the fungal cell, the RNA polymerase and the guide-RNA encoding polynucleotide operably linked to the promoter as defined in any one of claims 1-11.

13. A fungal cell comprising at least the RNA polymerase and the guide-RNA encoding polynucleotide operably linked to the promoter as defined in anyone of claims 1 -11, said fungal cell preferably being capable of producing a compound of interest, wherein the fungal cell is a yeast cell or a filamentous fungus cell.

14. A method for the production of a compound of interest comprising culturing the cell according to claim 13 under conditions conducive to the production of the compound of interest and, optionally, purifying or isolating said compound of interest.

## Patentansprüche

1. Verwendung einer viralen DNA-abhängigen Eine-Untereinheit-RNA-Polymerase, ausgewählt aus einer T3-, SP6-, K11- oder T7-RNA-Polymerase, für die Expression, innerhalb einer Pilzzelle, einer guide-RNA für ein RNA-guided-Nuklease-System, wobei die guide-RNA von einem Polynukleotid codiert wird, das funktionell mit einem virale-DNA-abhängige-Eine-Untereinheit-RNA-Polymerase-Promotor, ausgewählt aus einem T3-, SP6-, K11- oder T7-RNA-Polymerase-Promotor, verknüpft ist, und wobei die Pilzzelle eine Hefezelle oder eine Fadenpilzzelle ist.

2. Verwendung nach Anspruch 1, wobei die Pilzzelle eine aus der aus *Candida, Hansenula, Issatchenkia, Kluyveromyces*, *Pichia, Saccharomyces, Schizosaccharomyces, Yarrowia* und *Zygosaccharomyces* bestehenden Gruppe ausgewählte Hefezelle; vorzugsweise eine Fadenpilzzelle ist, die aus einer aus der aus *Acremonium, Aspergillus, Chrysosporium, Myceliophthora, Penicillium, Talaromyces, Rasamsonia, Thielavia, Fusarium* und *Trichoderma* bestehenden Gruppe ausgewählten Gattung stammt.

3. Verwendung nach Anspruch 1 oder 2, wobei die guide-RNA von einem linearen Nukleinsäurekonstrukt, von einem Genom oder von einem Vektor, vorzugsweise einem Plasmid, exprimiert wird.

4. Verwendung nach einem der Ansprüche 1-3, wobei mehrere verschiedene guide-RNAs entweder von einem einzigen DNA-abhängige-Eine-Untereinheit-RNA-Polymerase-Promotor, ausgewählt aus einem T3-, SP6-, K11- oder T7-RNA-Polymerase-Promotor, oder von mehreren DNAabhängige-Eine-Untereinheit-RNA-Polymerase-Promotoren, ausgewählt aus einem T3-, SP6-, K11- oder T7-RNA-Polymerase-Promotor, exprimiert werden.

5. Verwendung nach einem der Ansprüche 1-4, wobei die guide-RNA von einem oder mehreren DNA-abhängige-Eine-Untereinheit-RNA-Polymerase-Promotoren aus einer Bibliothek von DNA-abhängige-Eine-Untereinheit-RNA-Polymerase-Promotoren, ausgewählt aus einem T3-, SP6-, K11- oder T7-RNA-Polymerase-Promotor, exprimiert wird.

6. Verwendung nach einem der Ansprüche 1-5, wobei der Zelle eine NHEJ(Non-Homologous End Joining)-Komponente fehlt.

7. Verfahren zur Expression, innerhalb einer Pilzzelle, einer guide-RNA für ein RNA-guided-Nuklease-System, wobei die guide-RNA von einem Polynukleotid codiert wird, das funktionell mit einem virale-DNAabhängige-Eine-Untereinheit-RNA-Polymerase-Promotor, ausgewählt aus einem T3-, SP6-, K11- oder T7-RNA-Polymerase-Promotor, verknüpft ist, wobei die Transkription der guide-RNA durch eine virale DNAabhängige Eine-Untereinheit-RNA-Polymerase, ausgewählt aus einer T3-, SP6-, K11- oder T7-RNA-Polymerase, erfolgt und wobei die Pilzzelle eine Hefezelle oder eine Fadenpilzzelle ist.

8. Verfahren nach Anspruch 7, wobei die Pilzzelle eine aus der aus *Candida, Hansenula, Issatchenkia, Kluyveromyces*, *Pichia, Saccharomyces, Schizosaccharomyces, Yarrowia* und *Zygosaccharomyces* bestehenden Gruppe ausgewählte Hefezelle; vorzugsweise eine Fadenpilzzelle ist, die aus einer aus der aus *Acremonium, Aspergillus, Chrysosporium, Myceliophthora, Penicillium, Talaromyces, Rasamsonia, Thielavia, Fusarium* und *Trichoderma* bestehenden Gruppe ausgewählten Gattung stammt.

9. Verfahren nach einem der Ansprüche 7-8, wobei mehrere verschiedene guide-RNAs entweder von einem einzigen DNA-abhängige-Eine-Untereinheit-RNA-Polymerase-Promotor, ausgewählt aus einem T3-, SP6-, K11- oder T7-RNA-Polymerase-Promotor, oder von mehreren DNAabhängige-Eine-Untereinheit-RNA-Polymerase-Promotor, ausgewählt aus einem T3-, SP6-, K11- oder T7-RNA-Polymerase-Promotor, exprimiert werden.

10. Verfahren nach einem der Ansprüche 7-9, wobei die guide-RNA von einem oder mehreren DNA-abhängige-Eine-Untereinheit-RNA-Polymerase-Promotoren aus einer Bibliothek von DNA-abhängige-Eine-Untereinheit-RNA-Polymerase-Promotoren, ausgewählt aus einem T3-, SP6-, K11- oder T7-RNA-Polymerase-Promotor, exprimiert wird.

11. Verfahren nach einem der Ansprüche 7 bis 10, wobei der Zelle eine NHEJ(Non-Homologous End Joining)-Komponente fehlt.

12. Zusammensetzung, umfassend die Pilzzelle, die RNA-Polymerase und das die guide-RNA codierende, funktionell mit dem Promotor verknüpfte Polynukleotid nach einem der Ansprüche 1-11.

13. Pilzzelle, umfassend mindestens die RNA-Polymerase und das die guide-RNA codierende, funktionell mit dem Promotor verknüpfte Polynukleotid nach einem der Ansprüche 1-11, wobei die Pilzzelle vorzugsweise eine Verbindung von Interesse herstellen kann, wobei die Pilzzelle eine Hefezelle oder eine Fadenpilzzelle ist.

14. Verfahren zur Herstellung einer Verbindung von Interesse, umfassend das Kultivieren der Zelle nach Anspruch 13 unter Bedingungen, die zur Herstellung der Verbindung von Interesse führen, und optional Reinigen oder Isolieren der Verbindung von Interesse.

## Revendications

1. Utilisation d'une ARN polymérase dépendante de l'ADN à une seule sous-unité virale choisie parmi une ARN polymérase T3, SP6, K11 ou T7, pour l'expression à l'intérieur d'une cellule fongique d'un ARN-guide pour un système de nucléase guidée par l'ARN, dans laquelle l'ARN-guide est codé par un polynucléotide qui est lié de manière opérationnelle à un promoteur d'ARN polymérase dépendante de l'ADN à une seule sous-unité virale choisi parmi un promoteur d'ARN polymérase T3, SP6, K11 ou T7 et dans laquelle la cellule fongique est une cellule de levure ou une cellule de champignon filamenteux.

2. Utilisation selon la revendication 1, dans laquelle la cellule fongique est une cellule de levure sélectionnée dans le groupe constitué par *Candida, Hansenula, Issatchenkia, Kluyveromyces*, *Pichia, Saccharomyces, Schizosaccharomyces, Yarrowia* et *Zygosaccharomyces ;* de préférence, une cellule de champignon filamenteux appartenant à un genre choisi dans le groupe constitué par *Acremonium, Aspergillus, Chrysosporium, Myceliophthora, Pénicillium, Talaromyces, Rasamsonia, Thielavia, Fusarium* et *Trichoderma.*

3. Utilisation selon la revendication 1 ou 2, dans laquelle l'ARN-guide est exprimé à partir d'une construction linéaire d'acide nucléique, d'un génome ou d'un vecteur, de préférence un plasmide.

4. Utilisation selon l'une quelconque des revendications 1 à 3, dans laquelle plusieurs ARN-guides distincts sont exprimés à partir d'un seul promoteur d'ARN polymérase dépendante de l'ADN à une seule sous-unité sélectionné parmi un promoteur d'ARN polymérase T3, SP6, K11 ou T7 ou à partir de plusieurs promoteurs d'ARN polymérase dépendante de l'ADN à une seule sous-unité sélectionnés parmi un promoteur d'ARN polymérase T3, SP6, K11 ou T7.

5. Utilisation selon l'une quelconque des revendications 1 à 4, dans laquelle l'ARN-guide est exprimé à partir d'un ou de plusieurs promoteurs d'ARN polymérase dépendante de l'ADN à une seule sous-unité provenant d'une bibliothèque de promoteurs d'ARN polymérase dépendante de l'ADN à une seule sous-unité choisis parmi un promoteur d'ARN polymérase T3, SP6, K11 ou T7.

6. Utilisation selon l'une quelconque des revendications 1 à 5, dans laquelle la cellule est déficiente en un composant NHEJ (jonction d'extrémités non-homologues).

7. Procédé d'expression à l'intérieur d'une cellule fongique d'un ARN-guide pour un système de nucléase guidée par l'ARN, dans lequel l'ARN-guide est codé par un polynucléotide lié de manière opérationnelle à un promoteur d'ARN polymérase dépendante de l'ADN à une seule sous-unité virale choisi parmi un promoteur d'ARN polymérase T3, SP6, K11 ou T7, dans lequel la transcription de l'ARN-guide est effectuée par une ARN polymérase dépendante de l'ADN à une seule sous-unité virale, choisie parmi une ARN polymérase T3, SP6, K11 ou T7, et dans lequel la cellule fongique est une cellule de levure ou une cellule de champignon filamenteux.

8. Procédé selon la revendication 7, dans lequel la cellule fongique est une cellule de levure choisie dans le groupe constitué de *Candida, Hansenula, Issatchenkia, Kluyveromyces*, *Pichia, Saccharomyces, Schizosaccharomyces, Yarrowia* et *Zygosaccharomyces ;* de préférence, une cellule de champignon filamenteux appartenant à un genre choisi dans le groupe constitué par *Acremonium, Aspergillus, Chrysosporium, Myceliophthora, Penicillium, Talaromyces, Rasamsonia, Thielavia, Fusarium* et *Trichoderma.*

9. Procédé selon l'une quelconque des revendications 7 à 8, dans lequel plusieurs ARN-guides distincts sont exprimés à partir d'un seul promoteur d'ARN polymérase dépendante de l'ADN à une seule sous-unité sélectionné parmi un promoteur d'ARN polymérase T3, SP6, K11 ou T7, ou à partir de plusieurs promoteurs d'ARN polymérase dépendante de l'ADN à une seule sous-unité sélectionnés parmi un promoteur d'ARN polymérase T3, SP6, K11 ou T7.

10. Procédé selon l'une quelconque des revendications 7 à 9, dans lequel l'ARN-guide est exprimé à partir d'un ou de plusieurs promoteurs d'ARN polymérase dépendante de l'ADN à une seule sous-unité provenant d'une bibliothèque de promoteurs d'ARN polymérase dépendante de l'ADN à une seule sous-unité choisis parmi un promoteur d'ARN polymérase T3, SP6, K11 ou T7.

11. Procédé selon l'une quelconque des revendications 7 à 10, dans laquelle la cellule est déficiente en un composant NHEJ (jonction d'extrémités non-homologues).

12. Composition comprenant la cellule fongique, l'ARN polymérase et le polynucléotide codant pour l'ARN-guide lié de manière opérationnelle au promoteur tel que défini dans l'une quelconque des revendications 1 à 11.

13. Cellule fongique comprenant au moins l'ARN polymérase et le polynucléotide codant pour l'ARN-guide lié de manière fonctionnelle au promoteur tel que défini dans l'une quelconque des revendications 1 à 11, ladite cellule fongique étant de préférence capable de produire un composé d'intérêt, la cellule fongique étant une cellule de levure ou une cellule de champignon filamenteux.

14. Procédé de production d'un composé d'intérêt comprenant la culture de la cellule selon la revendication 13 dans des conditions propices à la production du composé d'intérêt et, éventuellement, la purification ou l'isolement dudit composé d'intérêt.
